# EUROPEAN PATENT SPECIFICATION

(11) **EP 2 600 839 B1**
(45) Date of publication and mention of the grant of the patent: **10.01.2018**
(21) Application number: 11746175.6
(22) Date of filing: 04.08.2011
(51) Int. Cl.: A61K 9/14, A61K 9/48, A61K 31/407

(54) **PHARMACEUTICAL DOSAGE FORM COMPRISING 6'-FLUORO-(N-METHYL- OR N,N-DIMETHYL-)-4-PHENYL-4',9'-DIHYDRO-3'H-SPIRO[CYLOHEXANE-1,1'-PYRANO[3,4,B]INDOL]-4-AMINE**
DARREICHUNGSFORM MIT 6'-FLUOR-(N-METHYL- ODER N,N-DIMETHYL)-4-PHENYL-4',9'-DIHYDRO-3'H-SPIRO[CYCLOHEXAN-1,1'-PYRANO[3,4,B]INDOL]-4-AMIN
FORME PHARMACEUTIQUE COMPRENANT UNE 6'-FLUORO-(N-MÉTHYL- OU N,N-DIMÉTHYL-)-4-PHÉNYL-4',9'-DIHYDRO-3'H-SPIRO[CYCLOHEXANE-1,1'-PYRANO[3,4,B]INDOL]-4-AMINE

(30) Priority: 04.08.2010 US 201161370643 P; 04.08.2010 EP 10008116
(43) Date of publication of application: 12.06.2013
(73) Proprietor: Grünenthal GmbH, 52078 Aachen (DE)
(72) Inventor: SCHILLER, Marc, 52076 Aachen (DE); GRÜNING, Nadja, 52062 Aachen (DE); FRIEDRICH, Ingo, 52078 Aachen (DE); KIRBY, Chris, Reading RG2 0NH (GB)
(86) International application number: PCT/EP2011/003909
(87) International publication number: WO 2012/016699

(56) References cited:
- WO-A1-2004/043967
- WO-A1-2006/082099
- WO-A1-2008/040481
- US-A1- 2007 048 228

## Description

### FIELD OF THE INVENTION

The invention relates to a pharmaceutical dosage form for preferably oral administration twice daily, once daily or less frequently, which contains a pharmacologically active agent according to general formula (I) 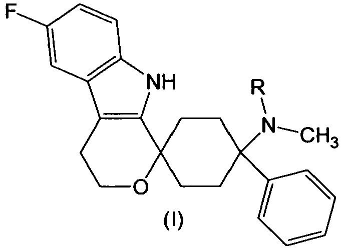 wherein R is -H or -CH₃, or a physiologically acceptable salt thereof.

The pharmacologically active agents according to general formula (I) can also be referred to as 6'-Fluoro-(N-methyl- or N,N-dimethyl-)-4-phenyl-4',9'-dihydro-3'H-spiro[cyclohexane-1,1'-pyrano[3,4,b]indol]-4-amine. Unless expressly stated otherwise, this term also includes the physiologically acceptable salts.

### BACKGROUND OF THE INVENTION

The pharmacologically active agents according to the invention are known from the prior art and can be administered orally, perorally, parenterally, intravenously, intraperitoneally, intradermally, intramuscularly, intranasally, buccally, rectally or locally, for example to the skin, the mucous membranes or into the eyes. The compounds exhibit analgesic properties and are particularly suitable for the treatment of acute, visceral, neuropathic or chronic pain (cf., e.g., WO 2004/043967 and WO 2008/040481).

Conventional analgesics are typically available as formulations providing immediate release or as formulations providing prolonged release.

On the one hand, formulations providing immediate release upon oral administration have the advantage that they lead to a fast release of the analgesic in the gastrointestinal tract. As a result, a comparatively high dose of the analgesic is quickly absorbed leading to high plasma levels within a short period of time and resulting in a rapid onset of pain relief, i.e. analgesic action begins shortly after administration. This is particularly desirable in acute pain.

At the same time, however, a rapid reduction in the analgesic action is usually observed, because metabolization and/or excretion of the analgesic cause a decrease of its plasma levels. For that reason, formulations providing immediate release of analgesics typically need to be administered frequently, e.g. eight times per day. This is not only detrimental with respect to patient compliance but also may cause comparatively high peak plasma drug concentrations and high fluctuations between peak and trough plasma drug concentrations which in turn may deteriorate tolerability.

On the other hand, formulations providing prolonged release upon oral administration have the advantage that they need to be administered less frequently, typically once daily or twice daily. This improves patient compliance and also can reduce peak plasma drug concentrations and fluctuations between peak and trough plasma drug concentrations which in turn may improve tolerability.

At the same time, however, release of the analgesic in the gastrointestinal tract is prolonged. As a result, a comparatively low dose of the analgesic is quickly absorbed leading to low plasma levels and resulting in a retarded onset of pain relief, i.e. analgesic action begins quite a while after first administration.

Furthermore, as formulations providing prolonged release typically contain higher doses of the analgesics than formulations providing immediate release, they bear a higher risk of being misused. Older patients in particular frequently have difficulties in taking solid pharmaceutical dosage forms. To counter this problem, various apparatuses have been developed by means of which solid pharmaceutical dosage forms may be comminuted or pulverized ("tablet crushers"). Such apparatuses are used, for example, by the care staff in old people's homes. The pharmaceutical dosage forms are then administered to the people being cared for not as tablets etc. but rather as powder, for example to get round the difficulties involved in swallowing tablets. However, the comminution of pharmaceutical dosage forms with such apparatuses is problematic if the pharmaceutical dosage forms are prolonged release formulations. As a rule, comminution then results in destruction of the inner structure of the pharmaceutical dosage form, which is responsible for the prolonged release, so doing away with the prolonged-release action. Consequently, after administration, frequently all the physiologically active substance originally contained in the pharmaceutical dosage form is released in a relatively short time, whereby a comparatively very high plasma concentration of the substance is abruptly reached for a relatively short period (dose dumping). In this way, the original prolonged-release formulations become immediate-release formulations. Depending on the physiological activity of the substance, this may cause considerable side-effects however, and in extreme cases may even lead to the death of the patient (cf., e.g., J. E. Mitchell, Oral Pharmaceutical dosage forms That Should Not Be Crushed: 2000 Update, Hospital Pharmacy, 2000; H. Miller et al., To Crush or Not to Crush, Nursing 2000; R. Griffith et al., Tablet Crushing and the law: the implications for nursing; Prof. Nurse 2003). Intentional chewing of prolonged-release formulations may also lead to an overdose of the substance contained therein. Sometimes patients chew the pharmaceutical dosage forms deliberately, though often in ignorance of the type and purpose of a prolonged-release formulation, because they hope for a quicker effect.

Formulations providing a dual release mode, i.e. a combination of immediate release with prolonged release, are also known (cf., e.g., C.M. Lopez et al., Compressed Matrix Core Tablet as a Quick/Slow Dual-Component Delivery System Containing Ibuprofen, AAPS PharmSciTech 2007; 8(3), E1-E8). However, these formulations typically rely upon immediate-release units and prolonged-release units that are locally separated from one another and therefore, such pharmaceutical dosage forms can only be prepared by specific and costly methods.

It is an object of the invention to provide pharmaceutical dosage forms containing 6'-Fluoro-(N-methyl- or N,N-dimethyl-)-4-phenyl-4',9'-dihydro-3'H-spiro[cyclohexane-1,1'-pyrano[3,4,b]-indol]-4-amine which have advantages compared to the pharmaceutical dosage forms of the prior art. In particular, the pharmaceutical dosage forms should provide good bioavailability and rapid pain relief already after the first administration, but also should have a high tolerability, good compliance, and safety.

This object has been achieved by the subject-matter of the patent claims.

It has been surprisingly found that 6'-Fluoro-(N-methyl- or N,N-dimethyl-)-4-phenyl-4',9'-dihydro-3'H-spiro[cyclohexane-1,1'-pyrano[3,4,b]indol]-4-amine has a comparatively poor water solubility. Further, it has been surprisingly found that in spite of said poor water solubility, pharmaceutical dosage forms can be prepared which provide immediate release of 6'-Fluoro-(N-methyl- or N,N-dimethyl-)-4-phenyl-4',9'-dihydro-3'H-spiro[cyclohexane-1,1'-pyrano[3,4,b]indol]-4-amine and provide good bioavailability. Still further, it has been surprisingly found that 6'-Fluoro-(N-methyl- or N,N-dimethyl-)-4-phenyl-4',9'-dihydro-3'H-spiro[cyclohexane-1,1'-pyrano[3,4,b]indol]-4-amine has a relatively large pharmacokinetic half life time (t_{1/2}) and thus, provides pharmacological activity for a comparatively extended period of time after administration.

Therefore, it has been surprisingly found that upon preferably oral administration of the pharmaceutical dosage form containing the pharmacologically active agent according to the invention, a rapid onset of pain relief can be achieved followed by a prolonged analgesic effect, although, or even if, the pharmaceutical dosage form provides immediate release. Therefore, the pharmaceutical dosage form according to the invention combines the advantageous properties of conventional formulations providing immediate release - rapid pain relief due to adequately high concentration of active ingredient just after administration of the pharmaceutical composition - with the advantageous properties of conventional formulations providing prolonged release - long-lasting analgesic action owing to an adequately high level of active ingredient over a prolonged time -, and at the same time even overcomes the drawbacks of said conventional formulations. By taking the pharmacologically active agent in the formulation according to the invention, the patient can effectively combat his pain acutely and, at the same time, treat if effectively over a prolonged period without further measures and merely by regular administration at 12 (or e.g., 24) hourly intervals.

It is particularly surprising that the pharmaceutical dosage form according to the invention not only allows the pharmacologically active agent to start flowing rapidly in the plasma when the pharmaceutical dosage form is first administered, leading to a rapid onset of pain relief in the patient owing to the immediate release, but at the same time ensures long-lasting therapeutic efficacy over a relatively long period (at least 12 hours). Therefore, the pain suffered by a patient can rapidly be alleviated when the pharmaceutical dosage form according to the invention is administered without the analgesic action quickly fading again.

The pharmaceutical dosage form according to the invention has good patient compliance and safety. Even if the pharmaceutical dosage form according to the invention is tampered with, e.g. by means of tablet crushers, dose dumping cannot occur - crushing the pharmaceutical dosage form does not further accelerate the immediate release profile.

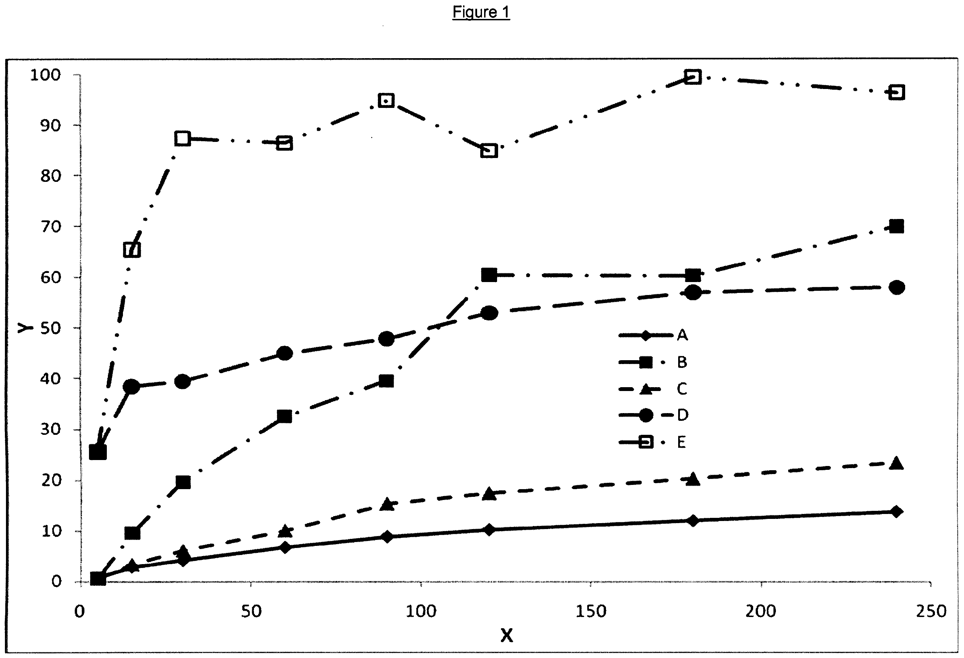

Figure 1 shows the release profile of the pharmacologically active agent according to general formula (I'b) from the pure solid itself (A), from solid formulations containing the active agent and a polymer (Kollidon 90; B) and from solid solutions containing the active agent, a polymer (Kollidon 90 or Kollidon VA 64) and a surfactant (Pluronic F68 or Tween 80; C-E) in hydrochloric acid (0.1 N). The X-Axis refers to the time in minutes and the Y-Axis refers to the amount of dissolved active agent in percent in relation to the whole amount of active agent originally contained in the dosage form.
Figure 2 shows the release profile of the pharmacologically active agent according to general formula (I'b) from the pure solid itself (A), from a solid formulation containing the active agent and a polymer (Kollidon VA64; B) and from solid solutions containing the active agent, a polymer (Kollidon VA 64) and a surfactant (Tween 80; C) in hydrochloric acid (0.1 N). The X-Axis refers to the time in minutes and the Y-Axis refers to the amount of dissolved active agent in percent in relation to the whole amount of active agent originally contained in the dosage form.
Figure 3 shows the X-ray powder diffractogram of a solid solution containing the pharmacologically active agent according to general formula (I'b), polymer Kollidon VA64 and surfactant Tween 80 before being objected to a storage stability test.
Figure 4 shows the X-ray powder diffractogram of a solid solution containing the pharmacologically active agent according to general formula (I'b), polymer Kollidon VA64 and surfactant Tween 80 after 4 weeks of storage at 25 °C and 60 % relative humidity.
Figure 5 shows the X-ray powder diffractogram of a solid solution containing the pharmacologically active agent according to general formula (I'b), polymer Kollidon VA64 and surfactant Tween 80 after 4 weeks of storage at 30 °C and 65 % relative humidity.
Figure 6 shows the averaged numerical rating scale (NRS) values measured over a 24 hour period after administration of different single doses of the compound according to formula (I'b) (200, 400, 600 µg) compared to morphine and placebo in patients with acute post-operative pain following orthopedic surgery (bunionectomy).

The invention relates to a pharmaceutical dosage form containing a pharmacologically active agent according to general formula (I) 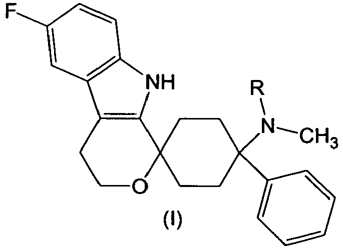 wherein R is -H or -CH₃,
or a physiologically acceptable salt thereof; said pharmaceutical dosage form being for administration twice daily, once daily or less frequently;
which releases in accordance with Ph. Eur. under in vitro conditions in 900 mL artificial gastric juice at pH 1.2 and 37 ± 0.5°C after 30 minutes according to the paddle method with sinker at 100 rpm at least 50 wt.-% of the pharmacologically active agent, based on the total amount of the pharmacologically active agent originally contained in the pharmaceutical dosage form; and
which comprises a solid polymeric matrix material in which the pharmacologically active agent according to general formula (I) is dispersed and immobilized in an amorphous or semi-amorphous state, and wherein the polymer is selected from the group consisting of polyvinylpyrrolidone, vinylpyrrolidone-polyvinylacetate copolymers, and any combinations thereof;
and wherein the relative weight ratio of the polymer related to the pharmacologically active agent according to general formula (I) is at least 6:1.

The pharmacologically active agent according to general formula (I) can also be referred to as "6'-fluoro-N-methyl-4-phenyl-4',9'-dihydro-3'H-spiro[cyclohexane-1,1'-pyrano[3,4,b]indol]-4-amine" when R is -H, and "6'-fluoro-N,N-dimethyl-4-phenyl-4',9'-dihydro-3'H-spiro-[cyclohexane-1,1'-pyrano[3,4,b]indol]-4-amine" when R is -CH₃; for the purpose of the specification, the pharmacologically active agent according to general formula (I) can also be referred to as "6'-fluoro-(N-methyl- or N,N-dimethyl-)-4-phenyl-4',9'-dihydro-3'H-spiro[cyclohexane-1,1'-pyrano[3,4,b]indol]-4-amine".

In a preferred embodiment, the pharmacologically active agent according to general formula (I) has a stereochemistry according to general formula (I') 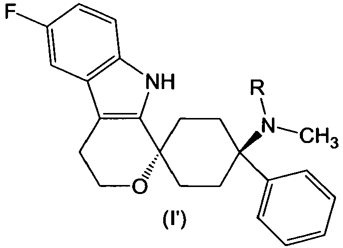 wherein R is -H or -CH₃, or a physiologically acceptable salt thereof.

In another embodiment of the pharmaceutical dosage form according to the invention, the compound of formula (I) is selected from 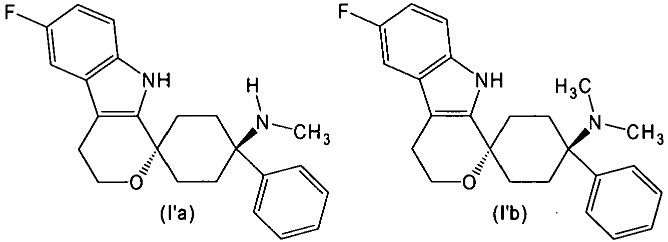 in the form of the free base or a physiologically acceptable salt thereof.

The free base according to general formula (I'a) can be systematically referred to as "1,1-(3-methylamino-3-phenylpentamethylene)-6-fluoro-1,3,4,9-tetrahydropyrano[3,4-b]indole (trans)" or as "(1r,4r)-6'-fluoro-N-methyl-4-phenyl-4',9'-dihydro-3'H-spiro[cyclohexane-1,1'-pyrano[3,4,b]indol]-4-amine", respectively.

The free base according to general formula (I'b) can be systematically referred to as "1,1-(3-dimethylamino-3-phenylpentamethylene)-6-fluoro-1,3,4,9-tetrahydropyrano[3,4-b]indole (trans)" or as "(1r,4r)-6'-fluoro-N,N-dimethyl-4-phenyl-4',9'-dihydro-3'H-spiro[cyclohexane-1,1'-pyrano[3,4,b]indol]-4-amine", respectively.

The definition of the pharmacologically active agent according to general formula (I) as used herein includes 6'-fluoro-(N-methyl- or N,N-dimethyl-)-4-phenyl-4',9'-dihydro-3'H-spiro-[cyclohexane-1,1'-pyrano[3,4,b]indol]-4-amine, derivatives thereof and stereoisomers thereof in any possible form, thereby particularly including solvates and polymorphs, salts, in particular acid addition salts and corresponding solvates and polymorphs.

In a preferred embodiment, the pharmacologically active agent according to general formula (I) is present as the single diastereomer according to general formula (I').

In another preferred embodiment the pharmacologically active agent according to general formula (I) is present as mixture of diastereomers. Such a mixture may contain the diastereomers in any ratio. A diastereomeric mixture could, for example, contain the diastereomers in a ratio of 60±5:40±5, 70±5:30±5, 80±5:20±5 or 90±5:10±5. Preferably, the pharmaceutical dosage form according to the invention contains the diastereomer according to general formula (I') in a diastereomeric excess (de) of at least 50%de, more preferably at least 60%de, still more preferably at least 70%de, yet more preferably at least 80%de, even more preferably at least 90%de, most preferably at least 95%de, and in particular at least 98%de, with respect to the other diastereomer (i.e. trans vs. cis and anti vs. syn, respectively).

6'-Fluoro-(N-methyl- or N,N-dimethyl-)-4-phenyl-4',9'-dihydro-3'H-spiro[cyclohexane-1,1'-pyrano[3,4,b]indol]-4-amine may be present in the pharmaceutical dosage form according to the invention in form of the free base or in form of an acid addition salt, whereby any suitable acid capable of forming such an addition salt may be used.

The conversion of 6'-Fluoro-(N-methyl- or N,N-dimethyl-)-4-phenyl-4',9'-dihydro-3'H-spiro-[cyclohexane-1,1'-pyrano[3,4,b]indol]-4-amine into a corresponding addition salt, for example, via reaction with a suitable acid may be effected in a manner well known to those skilled in the art. Suitable acids include but are not limited to hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid. Salt formation is preferably effected in a solvent, for example, diethyl ether, diisopropyl ether, alkyl acetates, acetone and/or 2-butanone. Moreover, trimethylchlorosilane in aqueous solution is also suitable for the preparation of hydrochlorides.

The pharmacologically active agent according to general formula (I) is contained in the pharmaceutical dosage form in a therapeutically effective amount. The amount that constitutes a therapeutically effective amount varies according to the compound, the condition being treated, the severity of said condition, the patient being treated, and whether the pharmaceutical dosage form is designed for an immediate or retarded release.

In a preferred embodiment, the content of the pharmacologically active agent according to the general formula (I) in the pharmaceutical dosage form according to the invention is at most 95 wt.-%, more preferably at most 50 wt.-%, yet more preferably at most 25 wt.-%, still more preferably at most 10 wt.-%, even more preferably at most 5 wt.%, most preferably at most 1.0 wt.%, and in particular at most 0.5 wt.-%.

In another preferred embodiment, the content of the pharmacologically active agent according to the general formula (I) in the pharmaceutical dosage form according to the invention is at least 0.001 wt.-%, more preferably at least 0.005 wt.%, yet more preferably at least 0.01 wt.-%, still more preferably at least 0.05 wt.-%, even more preferably at least 0.1 wt.-%, most preferably at least 0.5 wt.-%, and in particular at least 1.0 wt.-%.

Unless explicitly stated otherwise, in the meaning of the present invention the indication "wt.-%" shall mean weight of the respective ingredient per total weight of the pharmaceutical dosage form. In case that the pharmaceutical dosage form is film coated or encapsulated by an encapsulating medium which does not contain any amount of the pharmacologically active agent according to the general formula (I) and surrounds a core that in turn contains the total amount of the pharmacologically active agent according to the general formula (I), the indication "wt.-%" shall mean weight of the respective ingredient per total weight of the composition forming said core.

When the pharmaceutical dosage form is encapsulated or film coated, the pharmacologically active agent according to general formula (I) is preferably homogeneously distributed in the core of the pharmaceutical dosage form. Preferably, the encapsulating medium or film coating does not contain any pharmacologically active agent according to general formula (I).

The dose of the pharmacologically active agent according to general formula (I) preferably is in the range of 0.1 µg to 5000 µg, more preferably in the range of 0.1 µg to 1000 µg, and most preferably in the range of 1.0 µg to 100 µg or in the range of 30 µg to 600 µg.

In a preferred embodiment, the content of the pharmacologically active agent according to general formula (I) in the pharmaceutical dosage form is within the range of 25±20 µg, more preferably 25±15 µg, still more preferably 25±10 µg, and most preferably 25±5 µg.

In another preferred embodiment, the content of the pharmacologically active agent according to general formula (I) in the pharmaceutical dosage form is within the range of 40±35 µg, more preferably 40±30 µg, still more preferably 40±25 µg, yet more preferably 40±20 µg, even more preferably 40±15 µg, most preferably 40±10 µg, and in particular 40±5 µg.

In still another preferred embodiment, the content of the pharmacologically active agent according to general formula (I) in the pharmaceutical dosage form is within the range of 50±35 µg, more preferably 50±30 µg, still more preferably 50±25 µg, yet more preferably 50±20 µg, even more preferably 50±15 µg, most preferably 50±10 µg, and in particular 50±5 µg.

In yet another preferred embodiment, the content of the pharmacologically active agent according to general formula (I) in the pharmaceutical dosage form is within the range of 60±35 µg, more preferably 60±30 µg, still more preferably 60±25 µg, yet more preferably 60±20 µg, even more preferably 60±15 µg, most preferably 60±10 µg, and in particular 60±5 µg.

In another preferred embodiment, the content of the pharmacologically active agent according to general formula (I) in the pharmaceutical dosage form is within the range of 100±90 µg, more preferably 100±80 µg, still more preferably 100±60 µg, yet more preferably 100±40 µg, even more preferably 100±20 µg, most preferably 100±10 µg, and in particular 100±5 µg.

In still another preferred embodiment, the content of the pharmacologically active agent according to general formula (I) in the pharmaceutical dosage form is within the range of 200±175 µg, more preferably 200±150 µg, still more preferably 200±125 µg, yet more preferably 200±100 µg, even more preferably 200±75 µg, most preferably 200±50 µg, and in particular 200±25 µg.

In yet another preferred embodiment, the content of the pharmacologically active agent according to general formula (I) in the pharmaceutical dosage form is within the range of 400±350 µg, more preferably 400±300 µg, still more preferably 400±250 µg, yet more preferably 400±200 µg, even more preferably 400±150 µg, most preferably 400±100 µg, and in particular 400±50 µg.

In a preferred embodiment the pharmaceutical dosage form is for use in the treatment of acute pain, where the dose of the pharmacologically active agent according to general formula (I) preferably is in the range of 50 µg to 3000 µg, more preferably in the range of 100 µg to 1000 µg, even more preferably in the range of 300 µg to 500 µg, and most preferably in the range of 350 µg to 450 µg.

In another preferred embodiment, the pharmaceutical dosage form is for use in the treatment of acute pain, where the dose of the pharmacologically active agent according to general formula (I) preferably is in the range of 200 µg to 400 µg, and in particular in the range of 250 µg to 350 µg.

For the purpose of the specification, the wording "being for use in the treatment of pain" is equivalent with "being adapted for use in the treatment of pain".

In a preferred embodiment, the pharmaceutical dosage form is for use in the treatment of acute pain, where the dose of the pharmacologically active agent according to general formula (I) preferably is in the range of 200 µg to 400 µg, and in particular in the range of 250 µg to 350 µg.

In a preferred embodiment, the pharmaceutical dosage form is for use in the treatment of acute pain, where the dose of the pharmacologically active agent according to general formula (I) preferably is in the range of 250 µg to 450 µg, and in particular in the range of 300 µg to 400 µg.

In another preferred embodiment, the pharmaceutical dosage form is for use in the treatment of acute pain, where the dose of the pharmacologically active agent according to general formula (I) preferably is in the range of 300 µg to 500 µg, and in particular in the range of 350 µg to 450 µg.

In yet another preferred embodiment, the pharmaceutical dosage form is for use in the treatment of acute pain, where the dose of the pharmacologically active agent according to general formula (I) preferably is in the range of 350 µg to 550 µg, and in particular in the range of 400 µg to 500 µg.

In even another preferred embodiment, the pharmaceutical dosage form is for use in the treatment of acute pain, where the dose of the pharmacologically active agent according to general formula (I) preferably is in the range of 400 µg to 600 µg, and in particular in the range of 450 µg to 550 µg.

In another preferred embodiment the pharmaceutical dosage form is for use in the treatment of chronic pain, where the dose of the pharmacologically active agent according to general formula (I) preferably is in the range of 0.1 µg to 500 µg, more preferably in the range of 1 µg to 250 µg, even more preferably in the range of 5 µg to 100 µg, and most preferably in the range of 10 µg to 50 µg.

In a preferred embodiment, the pharmaceutical dosage form according to the invention is for oral administration, i.e. the pharmaceutical dosage form is adapted for oral administration. Suitable alternative pathways of administration of the pharmaceutical dosage form according to the invention include but are not limited to vaginal and rectal administration.

The pharmaceutical dosage form according to the invention is for administration twice daily, once daily or less frequently, i.e. the pharmaceutical dosage form is adapted for administration twice daily, once daily or less frequently.

In a preferred embodiment, the pharmaceutical dosage form according to the invention is for administration twice daily.

For the purpose of the specification, "administration twice daily" (bid) preferably means that the pharmaceutical dosage form is adapted for being administered according to a regimen comprising the administration of a first pharmaceutical dosage form according to the invention and the subsequent administration of a second pharmaceutical dosage form according to the invention, wherein both, the first and the second pharmaceutical dosage form are administered during a time interval of about 24 hours, but wherein the second pharmaceutical dosage form is administered not earlier than 6 hours, preferably not earlier than 8 hours, more preferably not earlier than 10 hours and in particular, about 12 hours after the first pharmaceutical dosage form has been administered.

In another preferred embodiment, the pharmaceutical dosage form according to the invention is for administration once daily.

For the purpose of the specification, "administration once daily" (sid) preferably means that the pharmaceutical dosage form is adapted for being administered according to a regimen comprising the administration of a first pharmaceutical dosage form according to the invention and the subsequent administration of a second pharmaceutical dosage form according to the invention, wherein both, the first and the second pharmaceutical dosage form are administered during a time interval of about 48 hours, but wherein the second pharmaceutical dosage form is administered not earlier than 18 hours, preferably not earlier than 20 hours, more preferably not earlier than 22 hours and in particular, about 24 hours after the first pharmaceutical dosage form has been administered.

In another preferred embodiment, the pharmaceutical dosage form according to the invention is for administration once daily or less frequently.

In another preferred embodiment, the pharmaceutical dosage form according to the invention is for administration less frequently than once daily, preferably thrice during four days (3/4), twice during three days (2/3), thrice during five days (3/5), once during two days (1/2), thrice in a week (3/7), twice during five days (2/5), once during three days (1/3), twice in a week (2/7), once during four days (1/4), once during five days (1/5), once during six days (1/6), or once in a week (1/7). According to this embodiment, administration once during two days (1/2) is particularly preferred.

A skilled person is fully aware that administration regimens "twice daily, once daily, or less frequently" may be realized by administering a single pharmaceutical dosage form containing the full amount of the pharmacologically active agent according to general formula (I) to be administered at a particular point in time or, alternatively, administering a multitude of dose units, i.e. two, three or more dose units, the sum of which multitude of dose units containing the full amount of the pharmacologically active agent according to general formula (I) to be administered at said particular point in time, where the individual dose units are for simultaneous administration or administration within a short period of time, e.g. within 5, 10 or 15 minutes.
Preferably, the pharmaceutical dosage form according to the invention provides immediate release of the pharmacologically active agent according to general formula (I). Preferably, the pharmaceutical dosage form is specifically designed to provide immediate release of the pharmacologically active agent according to general formula (I) *in vitro* in accordance with Ph. Eur. When the pharmaceutical dosage form is coated, e.g., with a coating that is soluble in gastric juice, the release kinetic is preferably monitored after such coating has been dissolved.
For the purpose of specification, the term "immediate release" refers to any release profile that fulfills at least one, preferably both, of the following requirements. First, the pharmaceutical dosage form disintegrates in 10 minutes or less following exposure to a disintegrating medium. Methods to determine the disintegration time are known to a person skilled in the art. For instance, they can be determined according to the USP XXIV disintegration test procedure, using, for example, an Erweka ZT-71 disintegration tester. Second, the pharmaceutical dosage form releases at least 70 wt.-% of the drug within 15 minutes following exposure to a dissolution medium. Preferably, the *in vitro* release properties of the pharmaceutical dosage form according to the invention are determined according to the paddle method with sinker at 50, 75 or 100 rpm, preferably under *in vitro* conditions at 37 ±0.5 °C in 900 mL artificial gastric juice at pH 1.2, or under the same conditions in non-artificial gastric juice. The pharmaceutical dosage form releases under *in vitro* conditions in 900 mL artificial gastric juice at pH 1.2 and 37 ±0.5 °C after 30 minutes according to the paddle method with sinker at 100 rpm at least 50 wt.-%, more preferably at least 60 wt.-%, still more preferably at least 70 wt.-%, yet more preferably at least 80 wt.-%, most preferably at least 90 wt.-%, and in particular at least 95 wt.-% of the pharmacologically active agent according to general formula (I), based on the total amount of the pharmacologically active agent according to general formula (I) originally contained in the pharmaceutical dosage form.

The pharmaceutical dosage form according to the invention exhibits excellent shelf-life and storage stability, i.e. neither the chemical composition, nor the physical characteristics, nor the dissolution profile of the pharmaceutical dosage form are altered significantly upon storage.

In a preferred embodiment, the pharmaceutical dosage form according to the invention provides sufficient stability to the pharmacologically active agent according to general formula (I) contained therein, so that after storage of the pharmaceutical dosage form at 40±2°C at 75% RH ±5% for a minimum time period of 6 weeks, preferably 3 months, the concentrations of undesirable degradants and impurities, respectively, preferably resulting from a degradation or decomposition of the pharmacologically active agent according to general formula (I) as such, is at most 1.0 wt.-%, more preferably at most 0.8 wt.-%, still more preferably at most 0.6 wt.-%, yet more preferably at most 0.4 wt.-%, even more preferably at most 0.2 wt.-%, most preferably at most 0.1 wt.-%, and in particular at most 0.05 wt.%, relative to the original content of the pharmacologically active agent according to general formula (I) in the pharmaceutical dosage form, i.e. its content before subjecting the pharmaceutical dosage form to storage.

It has been found that the pharmacologically active agent according to general formula (I) may be decomposed by elimination of the group -NRCH₃ thereby yielding 6'-fluoro-4-phenyl-4',9'-dihydro-3'H-spiro[cyclohex-3-ene-1,1'-pyrano[3,4-b]indole] which appears to be pharmacologically inactive. Preferably, after storage of the pharmaceutical dosage form at 40±2°C and 75% RH ±5%, or at 25±2°C and 60% RH ±5%, for a minimum time period of 6 weeks, preferably 3 months, the concentration of 6'-fluoro-4-phenyl-4',9'-dihydro-3'H-spiro[cyclohex-3-ene-1,1'-pyrano[3,4-b]indole] is at most 1.0 wt.%, more preferably at most 0.8 wt.-%, still more preferably at most 0.6 wt.-%, yet more preferably at most 0.4 wt.-%, even more preferably at most 0.2 wt.-%, most preferably at most 0.1 wt.-%, and in particular at most 0.05 wt.%, relative to the original content of the pharmacologically active agent according to general formula (I) in the pharmaceutical dosage form, i.e. its content before subjecting the pharmaceutical dosage form to storage.

A generally accepted accelerated test for the determination of a drug's stability according to ICH and FDA guidelines relates to the storage of a pharmaceutical formulation containing the drug (e.g., in its container and packaging). According to the ICH guidelines, a so-called accelerated storage testing should be conducted for pharmaceutical formulations at 40±2°C at 75% RH ±5% for a minimum time period of 6 months. Additionally, a so-called long-term storage testing should be conducted for pharmaceutical formulations at 25±2°C at not less than 60% RH ±5% for a minimum time period of 12 months. In case that all criteria have been met for the accelerated storage testing and long-term storage testing conditions during the 6-months period, the long-time storage testing may be shortened to 6 months and the corresponding data doubled to obtain estimated data for the 12-month period.

During the storage, samples of the pharmaceutical formulation are withdrawn at specified time intervals and analyzed in terms of their drug content, presence of impurities, their release profile and if applicable other parameters. According to the ICH guidelines, in all samples the purity of the drug should be ≥ 98%, the drug content should be 95-105% (FDA guideline: 90-110%). Furthermore, the pharmaceutical formulation should release >80% of the drug within 30 minutes.

In case of tablets and capsules that contain less than 50 mg of a drug, a content uniformity test should additionally be conducted for 10 randomly chosen dosage forms. The pharmaceutical formulation complies if none individual content is outside the limits of 85% to 115% of the average content. In case that an individual content is outside these limits, another 30 capsules have to be analyzed. The preparation fails to comply with the test if more than 3 individual contents are outside the limits of 85 to 115 % of the average content or if one or more individual contents are outside the limits of 75 % to 125 % of the average content.

In a preferred embodiment, after storage of the pharmaceutical dosage form for 6 months under long-term storage conditions (25°C and 60% relative humidity) in a sealed glass container, the degradation of the pharmacologically active agent according to general formula (I) does not exceed 2.0%, more preferably 1.5%, still more preferably 1.0%, and most preferably 0.5%.

In another preferred embodiment, after storage of the pharmaceutical dosage form for 6 months under accelerated storage conditions (40°C and 75% relative humidity) in a sealed glass container, the degradation of the pharmacologically active agent according to general formula (I) does not exceed 4%, more preferably 3%, still more preferably 2%, yet more preferably 1%, and most preferably 0.5%.

Preferably, after storage of the pharmaceutical dosage form for 6 months under long-term storage conditions (25°C and 60% relative humidity), the pharmaceutical dosage form releases under *in vitro* conditions in 900 mL artificial gastric juice at pH 1.2 and 37 ±0.5 °C after 30 minutes according to the paddle method with sinker at 100 rpm at least 50 wt.-%, more preferably at least 60 wt.-%, still more preferably at least 70 wt.-%, and most preferably at least 80 wt.-% of the pharmacologically active agent according to general formula (I), based on the total amount of the pharmacologically active agent according to general formula (I) originally contained in the pharmaceutical dosage form.

Preferably, after storage of the pharmaceutical dosage form for 6 months under accelerated storage conditions (40°C and 75% relative humidity), the pharmaceutical dosage form releases under *in vitro* conditions in 900 mL artificial gastric juice at pH 1.2 and 37 ±0.5 °C after 30 minutes according to the paddle method with sinker at 100 rpm at least 50 wt.-%, more preferably at least 60 wt.-%, still more preferably at least 70 wt.-%, and most preferably at least 80 wt.-% of the pharmacologically active agent according to general formula (I), based on the total amount of the pharmacologically active agent according to general formula (I) originally contained in the pharmaceutical dosage form.

The absorption properties of a pharmacologically active agent administered by a pharmaceutical dosage form can be described by the pharmacokinetic parameters Cₘₐₓ, tₘₐₓ and AUC₀₋ₜ. The determination of Cₘₐₓ and tₘₐₓ, as well as the calculation of an AUC are well known to a person skilled in the art and described, for example, in Bauer, Frömming, Führer, "Lehrbuch der Pharmazeutischen Technologie," 6th Edition (1999), and in Shargel, Wu-Pong, Yu, "Applied Biopharmaceuticals & Pharmacokinetics," 5th Edition (2005).

There is experimental evidence indicating that AUC₀₋ₜ and Cₘₐₓ of the pharmacologically active agent according to general formula (I) are proportional to the dose.

For the purpose of the specification, Cₘₐₓ is the highest plasma concentration of the pharmacologically active agent reached after single administration of the pharmaceutical dosage form.

For the purpose of the specification, tₘₐₓ is the time needed in order to reach Cₘₐₓ.

For the purpose of the specification, AUC₀₋ₜ is the area under the curve after single administration to the time t of the last sample that contained an analytically quantifiable concentration of the pharmacologically active agent.

For the purpose of the specification, AUC₀₋₇₂ₕ is the area under the curve baseline after single administration to 72 hours thereafter.

Preferably, the ratio Cₘₐₓ / dose is within the range of from 0.01 to 3.00 m⁻³, yet more preferably within the range of from 0.02 to 2.50 m⁻³, more preferably within the range of from 0.04 to 2.00 m⁻³, and most preferably within the range of from 0.06 to 1.69 m⁻³. In a preferred embodiment, the ratio Cₘₐₓ / dose is within the range of 0.40±0.35 m⁻³, more preferably 0.40±0.30 m⁻³, still more preferably 0.40±0.25 m⁻³, yet more preferably 0.40±0.20 m⁻³, even more preferably 0.40±0.15 m⁻³, most preferably 0.40±0.10 m⁻³, and in particular 0.40±0.05 m-³. In another preferred embodiment, the ratio Cₘₐₓ / dose is within the range of 0.80±0.70 m⁻³, more preferably 0.80±0.60 m⁻³, still more preferably 0.80±0.50 m⁻³, yet more preferably 0.80±0.40 m⁻³, even more preferably 0.80±0.30 m⁻³, most preferably 0.80±0.20 m⁻³, and in particular 0.80±0.10 m⁻³. In still another preferred embodiment, the ratio Cₘₐₓ / dose is within the range of 1.20±1.05 m⁻³, more preferably 1.20±0.90 m⁻³, still more preferably 1.20±0.75 m-³, yet more preferably 1.20±0.60 m⁻³, even more preferably 1.20±0.45 m⁻³, most preferably 1.20±0.30 m⁻³, and in particular 1.20±0.15 m⁻³.

Preferably, tₘₐₓ is within the range of from 15 minutes to 24 h, still more preferably within the range of from 20 minutes to 20 h, yet more preferably within the range of from 0.5 to 16 h, most preferably within the range of from 1 to 12 h, and in particular within the range of from 2 to 10 h. In a preferred embodiment, tₘₐₓ is within the range of 4±3.5 h, more preferably 4±3 h, still more preferably 4±2.5 h, yet more preferably 4±2 h, even more preferably 4±1.5 h, most preferably 4±1 h, and in particular 4±0.5 h. In another preferred embodiment, tₘₐₓ is within the range of 8±7 h, more preferably 8±6 h, still more preferably 8±5 h, yet more preferably 8±4 h, even more preferably 8±3 h, most preferably 8±2 h, and in particular 8±1 h. In still another preferred embodiment, tₘₐₓ is within the range of 12±11 h, more preferably 12±9 h, still more preferably 12±7 h, yet more preferably 12±5 h, even more preferably 12±3 h, most preferably 12±2 h, and in particular 12±1 h.

Preferably, the ratio AUC₀₋ₜ / dose is within the range from 0.3 to 20 h/m³, more preferably within the range of from 0.4 to 18 h/m³, still more preferably within the range of from 0.5 to 16.5 h/m³ and most preferably within the range of from 0.55 to 12.5 h/m³. In a preferred embodiment, the ratio AUC₀₋ₜ / dose is within the range of 3±2.5 h/m³, more preferably 3±2 h/m³, still more preferably 3±1.5 h/m³, yet more preferably 3±1 h/m³, even more preferably 3±0.75 h/m³, most preferably 3±0.5 h/m³, and in particular 3±0.25 h/m³. In another preferred embodiment, the ratio AUC₀₋ₜ / dose is within the range of 6±5 h/m³, more preferably 6±4 h/m³, still more preferably 6±3 h/m³, yet more preferably 6±2 h/m³, even more preferably 6±1.5 h/m³, most preferably 6±1 h/m³, and in particular 6±0.5 h/m³. In still another preferred embodiment, the ratio AUC₀₋ₜ / dose is within the range of 9±8 h/m³, more preferably 9±7 h/m³, still more preferably 9±5 h/m³, yet more preferably 9±4 h/m³, even more preferably 9±3 h/m³, most preferably 9±2 h/m³, and in particular 9±1 h/m³.
In a preferred embodiment, the pharmaceutical dosage form according to the invention is monolithic.
In another preferred embodiment, the pharmaceutical dosage form according to the invention comprises a core that is surrounded by a coating or by an encapsulating material. Disclosed is an embodiment, wherein the core is liquid and the pharmacologically active agent according to general formula (I) is dispersed, preferably dissolved in the liquid. Disclosed pharmaceutical dosage forms provide the pharmacologically active agent according to general formula (I) in form of self-(micro) emulsifying drug delivery systems, solid solutions, nanoparticles, cyclodextrin complexes, liposomes, micelles, micronized and/or amorphous states.

In general terms, disclosed options for formulation of poorly water-soluble drugs include crystalline solid, amorphous and lipid formulations.

Solid solutions according to the invention provide and sustain the pharmacologically active agent in an amorphous or semi-amorphous state immobilized in a polymer. Amorphous solutions may contain surfactants and polymers, thereby providing surface-activity during dispersion upon contact with water. Solid solutions can be formed using a variety of technologies such as spray drying and melt extrusion.

In a preferred embodiment, the pharmaceutical dosage form contains the pharmacologically active agent according to general formula (I) in form of a solid solution, i.e. molecularly dispersed in a solid matrix, so that preferably the pharmaceutical dosage form as such has amorphous or semi-amorphous nature. The solid solution preferably comprises the pharmacologically active agent according to general formula (I) in a molecular disperse form and an amorphous polymer matrix having a comparatively large specific surface. The pharmacologically active agent according to general formula (I) is preferably present in a molecular disperse form, i.e. the compound is truly solved and evenly spread in the solidified solution. The particle size of the compound is neither microcrystalline nor fine crystalline. The typical particle size is preferably from 0.1 - 1 µm.

Preferably, the pharmacologically active agent according to general formula (I) is molecularly dispersed in a matrix.
In a preferred embodiment, the pharmacologically active agent according to general formula (I) is molecularly dispersed in a non-crystalline matrix.
In another preferred embodiment, the pharmacologically active agent according to general formula (I) is molecularly dispersed in a non-amorphous matrix.
Preferably, the pharmacologically active agent according to general formula (I) is homogeneously distributed in the pharmaceutical dosage form according to the invention. The content of the pharmacologically active agent according to general formula (I) of two segments of the pharmaceutical dosage form having a volume of 1.0 mm³ each, deviate from one another by preferably not more than ±10%, more preferably not more than more than ±7.5%, still more preferably not more than ±5.0%, most preferably not more than ±2.5%, and in particular not more than ±1.0%. When the pharmaceutical dosage form is encapsulated or film-coated, said two segments of the pharmaceutical dosage form having a volume of 1.0 mm³ each are preferably segments of the core, i.e. do not contain any encapsulating medium or film coating, respectively.
Preferably, the pharmaceutical dosage form according to the invention is characterized by a comparatively homogeneous distribution of density. Preferably, the densities of two segments of the pharmaceutical dosage form having a volume of 1.0 mm³ each, deviate from one another by not more than ±10%, more preferably not more than more than ±7.5%, still more preferably not more than ±5.0%, most preferably not more than ±2.5%, and in particular not more than ±1.0%. When the pharmaceutical dosage form is encapsulated, said two segments of the pharmaceutical dosage form having a volume of 1.0 mm³ each are preferably segments of the core, i.e. do not contain any encapsulating medium or film coating.
In a preferred embodiment, the pharmaceutical dosage form further contains a surfactant.

For the purpose of the specification, the term "surfactant" refers to any compound that contains at least one hydrophobic group and at least one hydrophilic group. Preferably, the surfactant contains at least one terminal hydrophobic group (tail) and at least one terminal hydrophilic group (head).

The hydrophobic group is preferably selected from the group consisting of hydrocarbon, alkyl ether, fluorocarbon and siloxan groups.

In a preferred embodiment, the surfactant contains at least one aliphatic group comprising at least 3 carbon atoms, more preferably at least 4 carbon atoms, still more preferably at least 6 carbon atoms, yet more preferably 6 to 30 carbon atoms, and most preferably 8 to 24 carbon atoms. The aliphatic group may be a saturated or unsaturated, branched or unbranched (linear), terminal or internal aliphatic group.

Preferably, the surfactant contains at least one group derivable from a saturated or unsaturated fatty acid or from a saturated or unsaturated fatty alcohol, which group is preferably an ether, carboxylic acid ester or sulfuric acid ester group. Preferably, the saturated or unsaturated fatty acid or fatty alcohol contains at least 6 carbon atoms, yet more preferably 6 to 30 carbon atoms, and most preferably 8 to 24 carbon atoms.

In a preferred embodiment, the surfactant contains at least one group derivable from a saturated or unsaturated fatty acid, preferably C₆ to C₃₀ fatty acid, more preferably C₈ to C₂₄ fatty acid, and most preferably C₁₂ to C₂₂ fatty acid. Examples for suitable fatty acids are lauric acid, myristic acid, palmitic acid, stearic acid, arachidic acid, behenic acid, lignoceric acid, 12-hydroxystearic acid, oleic acid and ricinoleic acid.

In another preferred embodiment, the surfactant contains at least one group derivable from a saturated or unsaturated fatty alcohol, preferably C₆ to C₃₀ fatty alcohol, more preferably C₈ to C₂₄ fatty alcohol, and most preferably C₁₂ to C₂₂ fatty alcohol. Examples for suitable fatty alcohols are cetyl alcohol, stearyl alcohol, 2-octyldodecane-1-ol and 2-hexyldecane-1-ol.

Preferably, the surfactant has a molecular weight of at most 20,000 g/mol, more preferably at most 15,000 g/mol, still more preferably at most 10,000 g/mol, yet more preferably at most 5,000 g/mol, even more preferably at most 4,000 g/mol, most preferably at most 3,000 g/mol, and in particular within the range of from 100 g/mol to 2,500 g/mol.

Preferably, the surfactant is contained in a matrix in which the pharmacologically active agent according to general formula (I) is dispersed, preferably molecularly.

In a preferred embodiment, the pharmacologically active agent according to general formula (I) and the surfactant are intimately homogeneously distributed in a matrix so that the matrix does not contain any segments where either the pharmacologically active agent according to general formula (I) is present in the absence of the surfactant or where the surfactant is present in the absence of the pharmacologically active agent according to general formula (I).

In a preferred embodiment, the pharmaceutical dosage form contains a surfactant. In another preferred embodiment, the pharmaceutical dosage form contains a mixture of two or more surfactants.

In a preferred embodiment, the surfactant acts as an O/W emulsifier. In another preferred embodiment, the surfactant acts as a W/O emulsifier.

Preferably, the pharmaceutical dosage form contains a surfactant having a hydrophilic-lipophilic balance (HLB) of at least 10 or at least 11. More preferably, the hydrophilic-lipophilic balance (HLB) is at least 12 or at least 13. Most preferably, the hydrophilic-lipophilic balance (HLB) ranges within 14 and 16.

In another preferred embodiment, the hydrophilic-lipophilic balance (HLB) of the surfactant is at least 27, more preferably at least 29, still more preferably at least 31, yet more preferably at least 33, even more preferably at least 35, most preferably at least 37 and in particular at least 39.

Preferably, the hydrophilic-lipophilic balance (HLB) of the surfactant is at most 30, more preferably at most 28, still more preferably at most 26, yet more preferably at most 24, even more preferably at most 22, most preferably at most 20 and in particular at most 18.

In a preferred embodiment, the HLB value of the surfactant is within the range of 10±3.5, more preferably 10±3, still more preferably 10±2.5, yet more preferably 10±2, even more preferably 10±1.5, most preferably 10±1, and in particular 10±0.5. In another preferred embodiment, the HLB value of the surfactant is within the range of 12±3.5, more preferably 12±3, still more preferably 12±2.5, yet more preferably 12±2, even more preferably 12±1.5, most preferably 12±1, and in particular 12±0.5. In still another preferred embodiment, the HLB value of the surfactant is within the range of 14±3.5, more preferably 14±3, still more preferably 14±2.5, yet more preferably 14±2, even more preferably 14±1.5, most preferably 14±1, and in particular 14±0.5. In another preferred embodiment, the HLB value of the surfactant is within the range of 15±3.5, more preferably 15±3, still more preferably 15±2.5, yet more preferably 15±2, even more preferably 15±1.5, most preferably 15±1, and in particular 15±0.5. In yet another preferred embodiment, the HLB value of the surfactant is within the range of 16±3.5, more preferably 16±3, still more preferably 16±2.5, yet more preferably 16±2, even more preferably 16±1.5, most preferably 16±1, and in particular 16±0.5. In another preferred embodiment, the HLB value of the surfactant is within the range of 18±3.5, more preferably 18±3, still more preferably 18±2.5, yet more preferably 18±2, even more preferably 18±1.5, most preferably 18±1, and in particular 18±0.5.

The surfactant can be ionic, amphoteric or non-ionic.

In a preferred embodiment, the pharmaceutical dosage form contains an ionic surfactant, in particular an anionic surfactant.

Suitable anionic surfactants include but are not limited to sulfuric acid esters such as sodium lauryl sulfate (sodium dodecyl sulfate, e.g. Texapon® K12), sodium cetyl sulfate (e.g. Lanette E®), sodium cetylstearyl sulfate, sodium stearyl sulfate, sodium dioctylsulfosuccinate (docusate sodium); and the corresponding potassium or calcium salts thereof.

Preferably, the anionic surfactant has the general formula (II-a)

CₙH₂ₙ₊₁O-SO₃⁻M⁺ (II-a),

wherein n is an integer of from 8 to 30, preferably 10 to 24, more preferably 12 to 18; and M is selected from Li⁺, Na⁺, K⁺, NH₄⁺ 1/2 Mg²⁺ and 1/2 Ca²⁺.

Further suitable anionic surfactants include salts of cholic acid including sodium glycocholate (e.g. Konakion® MM, Cernevit®), sodium taurocholate and the corresponding potassium or ammonium salts.

In another preferred embodiment, the pharmaceutical dosage form contains a non-ionic surfactant. Suitable non-ionic surfactants include but are not limited to
- fatty alcohols that may be linear or branched, such as cetylalcohol, stearylalcohol, cetylstearyl alcohol, 2-octyldodecane-1-ol and 2-hexyldecane-1-ol;
- sterols, such as cholesterole;
- partial fatty acid esters of sorbitan such as sorbitanmonolaurate, sorbitanmonopalmitate, sorbitanmonostearate, sorbitantristearate, sorbitanmonooleate, sorbitansesquioleate and sorbitantrioleate;
- partial fatty acid esters of polyoxyethylene sorbitan (polyoxyethylene-sorbitan-fatty acid esters), preferably a fatty acid monoester of polyoxyethylene sorbitan, a fatty acid diester of polyoxyethylene sorbitan, or a fatty acid triester of polyoxyethylene sorbitan; e.g. mono- and tri- lauryl, palmityl, stearyl and oleyl esters, such as the type known under the name "polysorbat" and commercially available under the trade name "Tween" including Tween® 20 [polyoxyethylene(20)sorbitan monolaurate], Tween® 21 [polyoxyethylene(4)sorbitan monolaurate], Tween® 40 [polyoxyethylene(20)sorbitan monopalmitate], Tween® 60 [polyoxyethylene(20)sorbitan monostearate], Tween® 65 [polyoxyethylene(20)sorbitan tristearate], Tween® 80 [polyoxyethylene(20)sorbitan monooleate], Tween 81 [polyoxyethylene(5)sorbitan monooleate], and Tween® 85 [polyoxyethylene(20)sorbitan trioleate]; preferably a fatty acid monoester of polyoxyethylenesorbitan according to general formula (II-b) 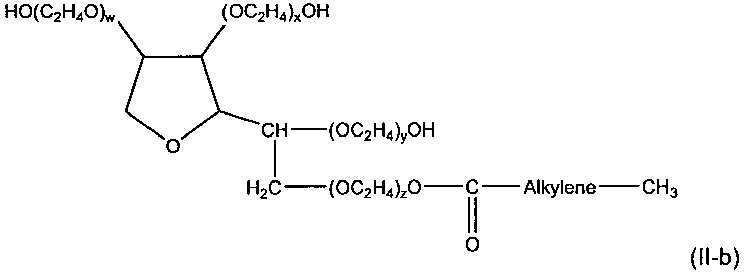 wherein (w+x+y+z) is within the range of from 15 to 100, preferably 16 to 80, more preferably 17 to 60, still more preferably 18 to 40 and most preferably 19 to 21; and alkylene is an optionally unsaturated alkylene group comprising 6 to 30 carbon atoms, more preferably 8 to 24 carbon atoms and most preferably 10 to 16 carbon atoms;
- polyoxyethyleneglycerole fatty acid esters such as mixtures of mono-, di- and triesters of glycerol and di- and monoesters of macrogols having molecular weights within the range of from 200 to 4000 g/mol, e.g., macrogolglycerolcaprylocaprate, macrogolglycerollaurate, macrogolglycerolococoate, macrogolglycerollinoleate, macrogol-20-glycerolmonostearate, macrogol-6-glycerolcaprylocaprate, macrogolglycerololeate; macrogolglycerolstearate, macrogolglycerolhydroxystearate (e.g. Cremophor® RH 40), and macrogolglycerolrizinoleate (e.g. Cremophor® EL);
- polyoxyethylene fatty acid esters, the fatty acid preferably having from about 8 to about 18 carbon atoms, e.g. macrogololeate, macrogolstearate, macrogol-15-hydroxystearate, polyoxyethylene esters of 12-hydroxystearic acid, such as the type known and commercially available under the trade name "Solutol HS 15"; preferably according to general formula (II-c)

   CH₃CH₂-(OCH₂CH₃)ₙ-O-CO-(CH₂)ₘCH₃ (II-c)

   wherein n is an integer of from 6 to 500, preferably 7 to 250, more preferably 8 to 100, still more preferably 9 to 75, yet more preferably 10 to 50, even more preferably 11 to 30, most preferably 12 to 25, and in particular 13 to 20; and
   wherein m is an integer of from 6 to 28; more preferably 6 to 26, still more preferably 8 to 24, yet more preferably 10 to 22, even more preferably 12 to 20, most preferably 14 to 18 and in particular 16;
- polyoxyethylene fatty alcohol ethers, e.g. macrogolcetylstearylether, macrogollarylether, macrogololeylether, macrogolstearylether;
- polyoxypropylene-polyoxyethylene block copolymers (poloxamers);
- fatty acid esters of saccharose; e.g. saccharose distearate, saccharose dioleate, saccharose dipalmitate, saccharose monostearate, saccharose monooleate, saccharose monopalmitate, saccharose monomyristate and saccharose monolaurate;
- fatty acid esters of polyglycerol, e.g. polyglycerololeate;
- polyoxyethylene esters of alpha-tocopheryl succinate, e.g. D-alpha-tocopheryl-PEG-1000-succinate (TPGS);
- polyglycolyzed glycerides, such as the types known and commercially available under the trade names "Gelucire 44/14", "Gelucire 50/13 and "Labrasol";
- reaction products of a natural or hydrogenated castor oil and ethylene oxide such as the various liquid surfactants known and commercially available under the trade name "Cremophor"; and
- partial fatty acid esters of multifunctional alcohols, such as glycerol fatty acid esters, e.g. mono- and tri-lauryl, palmityl, stearyl and oleyl esters, for example glycerol monostearate, glycerol monooleate, e.g. glyceryl monooleate 40, known and commercially available under the trade name "Peceol"; glycerole dibehenate, glycerole distearate, glycerole monolinoleate; ethyleneglycol monostearate, ethyleneglycol monopalmitostearate, pentaerythritol monostearate.

Especially preferred surfactants of this class that are contained in the pharmaceutical dosage form according to the invention are non-ionic surfactants having a hydrophilic-lipophilic balance (HLB) of at least 10, in particular non-ionic surfactants having an HLB value of at least 12, more in particular non-ionic surfactant's having an HLB value within 14 and 16. Examples for this type of surfactants are the above-listed surfactants "polysorbate 80" (Tween® 80) and "Solutol® HS 15".
Solutol® HS-15 is a mixture of polyethyleneglycol 660 12-hydroxystearate and polyethylene glycol. It is a white paste at room temperature that becomes liquid at about 30°C and has an HLB of about 15.
Tween® 80 [polyoxyethylene(20)sorbitan monooleate] is liquid at room temperature, has a viscosity of 375-480 mPa·s and has an HLB of about 15.
In a preferred embodiment, the content of the surfactant is at least 0.001 wt.-% or at least 0.005 wt.-%, more preferably at least 0.01 wt.-% or at least 0.05 wt.%, still more preferably at least 0.1 wt.-%, at least 0.2 wt.%, or at least 0.3 wt.%, yet more preferably at least 0.4 wt.%, at least 0.5 wt.%, or at least 0.6 wt.-%, and in particular at least 0.7 wt.-%, at least 0.8 wt.%, at least 0.9 wt.-%, or at least 1.0 wt.%, based on the total weight of the pharmaceutical dosage form.

In a particular preferred embodiment,
- the pharmaceutical dosage form contains a surfactant having a HLB value of at least 10 in an amount of at least 0.001 wt.-%, based on the total weight of the pharmaceutical dosage form; and/or
- the pharmaceutical dosage form contains 0.01 % to 95 % of the pharmacologically active agent (A); and/or
- the pharmaceutical dosage form has a weight within the range of from 0.1 mg to 2,000 mg; and/or
- the pharmaceutical dosage form contains a polymer with a molecular weight within the range of from 1,000 g/mol to 15 million g/mol; and/or
- the pharmaceutical dosage form is for oral administration; and/or
- the pharmaceutical dosage form contains the pharmacologically active agent according to general formula (I) in a dose of from 10 µg to 50 µg or of from 300 µg to 500 µg; and/or
- the pharmaceutical dosage form provides immediate release of the pharmacologically active agent according to general formula (I) *in vitro* in accordance with Ph. Eur.; and/or
- tₘₐₓ is within the range of from 0.5 to 16 h; and/or
- the ratio AUC₀₋ₜ/ dose is within the range of from 0.5 to 16.5 h/m³; and/or
- ratio Cₘₐₓ / dose is within the range of from 0.06 to 1.69 m⁻³.
The pharmaceutical dosage form according to the invention, particularly when it contains the pharmacologically active agent according to general formula (I) in form of a solid solution, i.e. molecularly dispersed in a solid matrix, further contains at least one matrix material. Said matrix material comprises a polymer selected from the group consisting of polyvinylpyrrolidone, vinylpyrrolidone-polyvinylacetate copolymers, and combinations thereof. Preferred examples of polyvinylpyrrolidone are commercialized as Kollidon® 90 and examples of vinylpyrrolidone-polyvinyl acetate copolymer are commercialized as Kollidon® VA64.
For the purpose of the specification, "molecularly dispersed in a solid matrix", e.g. in a polymer, means that a substantial portion of the overall content of the pharmacologically active agent according to general formula (I) is present in non-crystalline form, i.e. does not provide X-ray reflexes. Preferably, the content of non-crystalline pharmacologically active agent according to general formula (I) is at least 60 wt.-%, more preferably at least 65 wt.-%, still more preferably at least 70 wt.-%, yet more preferably at least 75 wt.-%, even more preferably at least 80 wt.-%, most preferably at least 85 wt.-%, and in particular at least 90 wt.-%, based on the total content of pharmacologically active agent according to general formula (I).

In a preferred embodiment the pharmaceutical dosage form according to the invention contains a polymer with a weight average molecular weight of preferably at least 50,000 g/mol, more preferably at least 100,000 g/mol, yet more preferably at least 250,000 g/mol, still more preferably at least 500,000 g/mol, most preferably at least 750,000 g/mol and in particularly at least 800,000 g/mol.

In another preferred embodiment the pharmaceutical dosage form according to the invention contains a polymer with a weight average molecular weight of preferably at least 5000 g/mol, more preferably at least 10,000 g/mol, yet more preferably at least 20,000 g/mol, still more preferably at least 30,000 g/mol, even more preferably at least 40,000 g/mol, most preferably at least 50,000 g/mol and in particular within the range of from 50,000 g/mol to 250,000 g/mol.

In a preferred embodiment, the pharmaceutical dosage form according to the invention contains the pharmacologically active agent according to general formula (I) in form of a solid solution, i.e. molecularly dispersed in a solid matrix, wherein the matrix comprises one or more polymers and wherein the content of the polymer(s) is within the range of 25±22.5 wt.-%, more preferably 25±20 wt.-%, still more preferably 25±17.5 wt.-%, yet more preferably 25±15 wt.%, even more preferably 25±12.5 wt.%, most preferably 25±10 wt.-% and in particular 25±7.5 wt.-%, based on the total weight of the pharmaceutical dosage form.

In another preferred embodiment, the pharmaceutical dosage form according to the invention contains the pharmacologically active agent according to general formula (I) in form of a solid solution, i.e. molecularly dispersed in a solid matrix, wherein the matrix comprises one or more polymers and wherein the content of the polymer(s) is within the range of 50±22.5 wt.-%, more preferably 50±20 wt.-%, still more preferably 50±17.5 wt.-%, yet more preferably 50±15 wt.-%, even more preferably 50±12.5 wt.-%, most preferably 50±10 wt.-% and in particular 50±7.5 wt.-%, based on the total weight of the pharmaceutical dosage form.

In still another preferred embodiment, the pharmaceutical dosage form according to the invention contains the pharmacologically active agent according to general formula (I) in form of a solid solution, i.e. molecularly dispersed in a solid matrix, wherein the matrix comprises one or more polymers and wherein the content of the polymer(s) is within the range of 75±22.5 wt.-%, more preferably 75±20 wt.-%, still more preferably 75±17.5 wt.-%, yet more preferably 75±15 wt.-%, even more preferably 75±12.5 wt.-%, most preferably 75±10 wt.-% and in particular 75±7.5 wt.-%, based on the total weight of the pharmaceutical dosage form. The pharmaceutical dosage form according to the invention contains a polymer which comprises repeating units derived from vinylpyrrolidones. In another preferred embodiment the polymer comprises monomer units derived from vinyl acetates. Preferably, the polymer is a copolymer comprising repeating units derived from vinylpyrrolidones and repeating units derived from vinyl acetates, wherein the weight ratio of repeating units derived from vinylpyrrolidones : repeating units derived from vinyl acetates is preferably at most 10:1, more preferably at most 4.5:1, still more preferably at most 4:1, most preferably at most 2:1 and in particular at most 1.5:1. The relative weight ratio of the polymer related to the pharmacologically active agent according to general formula (I) in the pharmaceutical dosage form according to the invention is at least 3:1 or at least 4:1, more preferably at least 5:1 or at least 6:1, still more preferably at least 7:1 or at least 8:1, yet more preferably at least 9:1 or at least 10:1, even more preferably at least 11:1 or at least 12:1, most preferably at least 13:1 or at least 14:1 and in particular at least 15:1 or at least 16:1. In a preferred embodiment the relative weight ratio of the polymer, especially of vinylpyrrolidone-vinyl acetate copolymer, related to the pharmacologically active agent according to general formula (I) in the pharmaceutical dosage form according to the invention ranges from 10:1 to 25:1 and in particular from 15:1 to 25:1.

In a preferred embodiment the pharmaceutical dosage form according to the invention, particularly when it contains the pharmacologically active agent according to general formula (I) in form of a solid solution, i.e. molecularly dispersed in a solid matrix, may further contain at least one surfactant selected from the group containing partial fatty acid esters of polyoxyethylene sorbitan (polyoxyethylene-sorbitan-fatty acid esters), preferably a fatty acid monoester of polyoxyethylene sorbitan, a fatty acid diester of polyoxyethylene sorbitan, or a fatty acid triester of polyoxyethylene sorbitan; sulfuric acid esters, or the alkali or earthalkali salts thereof; and poloxamers.

In a preferred embodiment the pharmaceutical dosage form according to the invention contains a surfactant with a weight average molecular weight of preferably at least 500 g/mol, more preferably at least 1,000 g/mol, yet more preferably at least 2,500 g/mol, still more preferably at least 5,000 g/mol, most preferably at least 7,000 g/mol and in particularly at least 8,000 g/mol.

In another preferred embodiment the pharmaceutical dosage form according to the invention contains a surfactant with a weight average molecular weight of preferably at least 100 g/mol, more preferably at least 250 g/mol, yet more preferably at least 500 g/mol, still more preferably at least 750 g/mol, most preferably at least 1,000 g/mol and in particularly at least 1,250 g/mol.

In another preferred embodiment the dosage form according to the invention contains a surfactant, preferably [polyoxyethylene(20)sorbitan monooleate] or polyoxyethylene-polyoxypropylene block co-polymer, in a content of preferably 0.5 wt-% to 80 wt-%, more preferably 1.5 wt-% to 60 wt-%, still more preferably 2.5 wt-% to 50 wt-%, yet more preferably 3.0 wt-% to 40 wt-%, most preferably 3.5 wt-% to 20 wt-%, and in particular 4 wt-% to 10 wt-%, based on the total weight of the pharmaceutical dosage form.

Preferred embodiment A¹ to A²⁰ of the pharmaceutical dosage form according to the invention are summarized in the table here below:

| embodiment | **A¹** | | **A²** | | **A³** | | **A⁴** | | **A⁵** | |
|---|---|---|---|---|---|---|---|---|---|---|
| ingredient | nature | ratio | nature | ratio | nature | ratio | nature | ratio | nature | ratio |
| pharmacologically active agent according to general formula (I) | X¹ | 1 | X¹ | 1 | X² | 1 | X² | 1 | X³ | 1 |
| polymer | Y¹ | 19±17 | Y¹ | 19±11 | Y² | 19±7 | Y² | 19±5 | Y³ | 19±3 |
| surfactant | Z¹ | 5±4 | Z¹ | 5±3 | Z² | 5±2 | Z² | 5±1 | Z³ | 5±0.5 |

| embodiment | **A⁶** | | **A⁷** | | **A⁸** | | **A⁹** | | **A¹⁰** | |
|---|---|---|---|---|---|---|---|---|---|---|
| ingredient | nature | ratio | nature | ratio | nature | ratio | nature | ratio | nature | ratio |
| pharmacologically active agent according to general formula (I) | X¹ | 2 | X¹ | 2 | X² | 2 | X² | 2 | X³ | 2 |
| polymer | Y¹ | 38±34 | Y¹ | 38±22 | Y² | 38±14 | Y² | 38±10 | Y³ | 38±6 |
| surfactant | Z¹ | 5±4 | Z¹ | 5±3 | Z² | 5±2 | Z² | 5±1 | Z³ | 5±0.5 |

| embodiment | **A¹¹** | | **A¹²** | | **A¹³** | | **A¹⁴** | | **A¹⁵** | |
|---|---|---|---|---|---|---|---|---|---|---|
| ingredient | nature | ratio | nature | ratio | nature | ratio | nature | ratio | nature | ratio |
| pharmacologically active agent according to general formula (I) | X¹ | 3 | X¹ | 3 | X² | 3 | X² | 3 | X³ | 3 |
| polymer | Y¹ | 57±51 | Y¹ | 57±33 | Y² | 57±21 | Y² | 57±15 | Y³ | 57±9 |
| surfactant | Z¹ | 5±4 | Z¹ | 5±3 | Z² | 5±2 | Z² | 5±1 | Z³ | 5±0.5 |

| embodiment | **A¹⁶** | | **A¹⁷** | | **A¹⁸** | | **A¹⁹** | | **A²⁰** | |
|---|---|---|---|---|---|---|---|---|---|---|
| ingredient | nature | ratio | nature | ratio | nature | ratio | nature | ratio | nature | ratio |
| pharmacologically active agent according to general formula (I) | X¹ | 4 | X¹ | 4 | X² | 4 | X² | 4 | X³ | 4 |
| polymer | Y¹ | 76±68 | Y¹ | 76±44 | Y² | 76±28 | Y² | 76±20 | Y³ | 76±12 |
| surfactant | Z¹ | 5±4 | Z¹ | 5±3 | Z² | 5±2 | Z² | 5±1 | Z³ | 5±0.5 |

wherein
nature refers to the chemical nature of the ingredient;
ratio refers to the relative weight proportion of the ingredient with respect to the other two ingredients;
- X¹: means the pharmacologically active agent according to general formula (I) or a physiologically acceptable salt thereof;
- X²: means the pharmacologically active agent according to general formula (I') or a physiologically acceptable salt thereof;
- X³: means (1r,4r)-6'-fluoro-N,N-dimethyl-4-phenyl-4',9'-dihydro-3'H-spiro[cyclohexane-1,1'-pyrano[3,4,b]indol]-4-amine, or (1r,4r)-6'-fluoro-N-methyl-4-phenyl-4',9'-dihydro-3'H-spiro[cyclohexane-1,1'-pyrano[3,4,b]indol]-4-amine, or a physiologically acceptable salt thereof;
- Y¹: means a polymer selected from the group consisting of polyvinylpyrrolidone, vinylpyrrolidone-vinylacetate copolymers, cellulose derivatives, preferably cellulose esters or cellulose ethers, polymethacrylates, polyethylene oxides, polyethylene glycols and any combinations thereof;
- Y²: means vinylpyrrolidone-vinylacetate copolymer;
- Y³: means vinylpyrrolidone-vinylacetate copolymers having a weight average molecular weight within the range of from 40,000 to 250,000 g/mol;
- Z¹: means a nonionic surfactan with a HLB value of 10-20;
- Z²: means a surfactant selected from the group of partial fatty acid esters of polyoxyethylene sorbitan;
- Z³: means a surfactant according to general forumla (II-b).

For example, according to the above table, embodiment A⁹ relates to a pharmacologically active agent according to general formula (I') or a physiologically acceptable salt thereof, a vinylpyrrolidone-polyvinylacetate copolymer and a surfactant selected from the group of polysorbitanes, wherein the weight ratio is 2:38:5.

A further aspect of the invention relates to the pharmaceutical dosage form according to the invention as described above for use in the treatment of pain.

A further aspect of the invention relates to a method of treating pain comprising the twice daily, once daily, or less frequently, preferably oral administration of the pharmaceutical dosage form according to the invention to a subject in need thereof.

Preferably, the pain is selected from acute, visceral, neuropathic or chronic pain.

### EXAMPLES

The following examples further illustrate the invention but are not to be construed as limiting its scope.

### General procedure for preparing the solid solution according to the invention

Active pharmaceutical ingredient (API), i.e. the pharmacologically active agent according to general formula (I), and a polymer were dispersed in dichloromethane in a flask. In some cases, a surfactant was added. The flask was heated and subjected to ultrasound in order to dissolve the ingredients properly. Where applicable, the solution was filtered through a sintered glass filter in order to remove traces of undissolved material. The solvent was evaporated by means of a rotary evaporator at a temperature of 60 °C. The solid residue was further dried under high vacuum overnight. The dried material was transferred to a sealed glass vial for the analysis for amorphous contents by using XRPD and DSC. The dissolution behavior was analyzed in 0.1 N HCl.

### Example 1:

Following the general procedure, solid solutions having the following compositions were prepared:

| Ex. | API | polymer | weight ratio API : polymer | surfactant | content surfactant |
|---|---|---|---|---|---|
| 1-A | + | - | - | - | - |
| 1-B | + | Kollidon 90 | 1:4 | - | - |
| 1-C | + | Kollidon 90 | 1:4 | Pluronic F68 | 5 wt.-% |
| 1-D | + | Kollidon VA 64 | 1:5 | Tween 80 | 5 wt.-% |
| 1-E | + | Kollidon VA 64 | 1:19 | Tween 80 | 5 wt.-% |

Examples 1-A to 1-D are not according to the invention.

Figure 1 shows the release profile of the API. The X-Axis refers to the time in minutes and the Y-Axis refers to the amount of dissolved API in percent in relation to the whole amount API originally contained in the dosage form.
It becomes evident from Figure 1 that the solid solution containing Kollidon VA64/Tween 80 in a ratio of 1:19 of the API and the polymer (example 1-E) showed a substantially higher dissolution rate than any of the other formulations (examples 1-A to 1-D). Nearly 90 % of the API was dissolved in the first 30 minutes. Pluronic F68 to the formulation with Kollidon 90 (1:4) resulted in a substantial decrease of the dissolution rate. Pluronic F68 is a difunctional block copolymer surfactant terminating in primary hydroxyl groups and having a HLB value of >24.

### Example 2:

Following the general procedure and in analogy to example 1, solid solutions having the following compositions were prepared:

| Ex. | API | polymer | weight ratio API : polymer | surfactant | content surfactant |
|---|---|---|---|---|---|
| 2-A | + | - | - | - | - |
| 2-B | + | Kollidon VA64 | 1:19 | - | - |
| 2-C | + | Kollidon VA64 | 1:19 | Tween 80 | 5 wt.-% |

Examples 2-A and 2-B are not according to the invention.

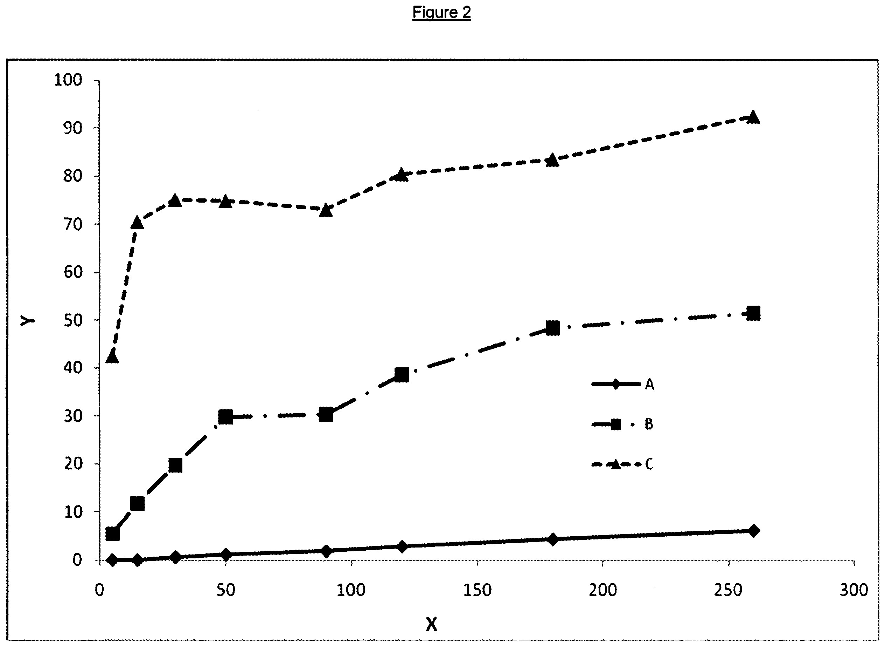

Figure 2 shows the release profile of the API. The X-Axis refers to the time in minutes and the Y-Axis refers to the amount of dissolved API in percent in relation to the whole amount API originally contained in the dosage form.
It becomes evident from Figure 2 that the formulation containing Tween 80 provided a faster release with a high extent of dissolution, while in the absence of the surfactant the dissolution rate was reduced; in the absence of surfactant (example 2-B), the overall extent of dissolution was only 55 % compared to the formulation containing Tween 80 (example 2-C).

### Example 3:

The storage stability of a solid solution was tested under various conditions and analyzed by using XRPD. The solid solution was prepared in accordance with examples 1 and 2 and had the following composition:

| Ex. | API | polymer | weight ratio API : polymer | surfactant | content surfactant |
|---|---|---|---|---|---|
| 3 | + | Kollidon VA64 | 1:19 | Tween 80 | 4 wt.-% |

Unit doses of the blended formulation were filled into size 0 hard gelatin capsules. The encapsulated formulations were stored at 25 °C and 60 % relative humidity (RH) at 30 °C and 65 % relative humidity, respectively. After predetermined time periods of 1, 2, and 4 weeks, the capsules were removed from the storage and analyzed.
The solid solution of the API, especially the formulation with API:V64 in a ratio of 1:19, revealed to be physically stable over a time period of 4 weeks at 25 °C and 30 °C demonstrated via XRPD.

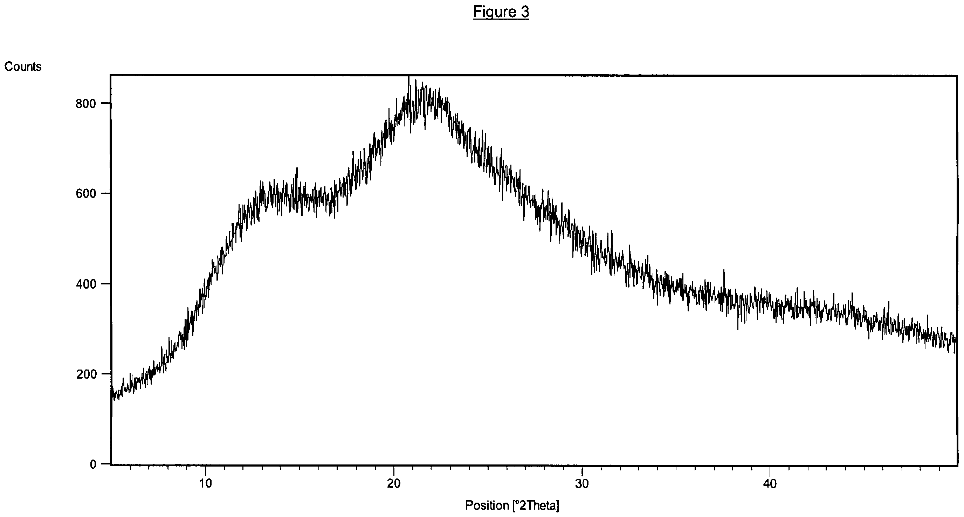

Figure 3 shows the XRPD result of the API solid solution at time zero.

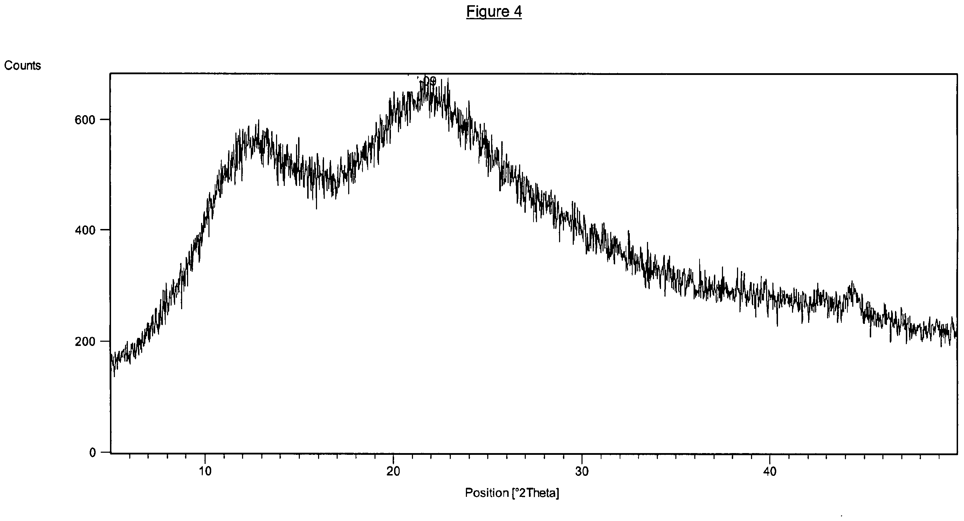

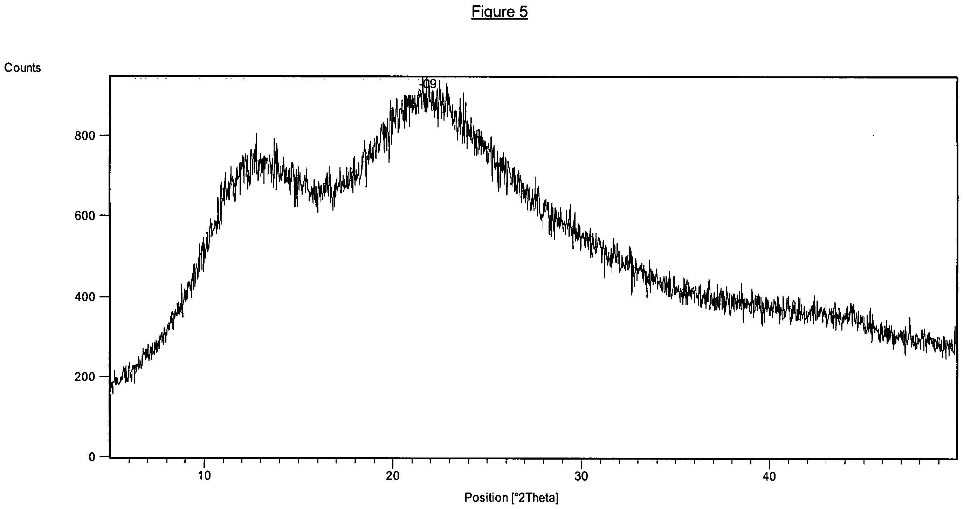

Figures 4 and 5 show the result after 4 weeks of storage at 25 °C and 60 % relative humidity and at 30 °C and 65 % relative humidity, respectively.
It is clear from Figures 3 to 5 that the drug product has amorphous or at least semi-amorphous nature.

### Reference example 4:

Clinical studies were conducted to determine the analgesic efficacy and tolerability of single doses of the compound according to formula (I'b) (200 µg, 400 µg and 600 µg, based on the amount of the free base; hemicitrate oral solution of compound (I'b) in Macrogol 400) compared to that of morphine (60 mg, controlled-released form) and placebo in patients with acute post-operative pain following orthopedic surgery (bunionectomy).
For this purpose, 258 patients of either sex were included in a randomized, placebo-controlled, double-blind clinical trial in parallel groups. Treatment groups were well-balanced with respect to demographics and baseline characteristics with a slight imbalance in baseline pain and ethnicity.

After surgery, all patients were initially treated with local post-operative anesthesia via a popliteal block. Due to different kinetics of the compound according to formula (I'b) and morphine, the patients were then treated with either one of the two drugs or with placebo at slightly different times:

One hour before the popliteal block was stopped, patients were randomized and part of them were dosed with a single dose of the compound according to formula (I'b) (200 µg, 400 µg or 600 µg) or placebo, while the others received morphine or placebo 2 hours after the popliteal block had been stopped.

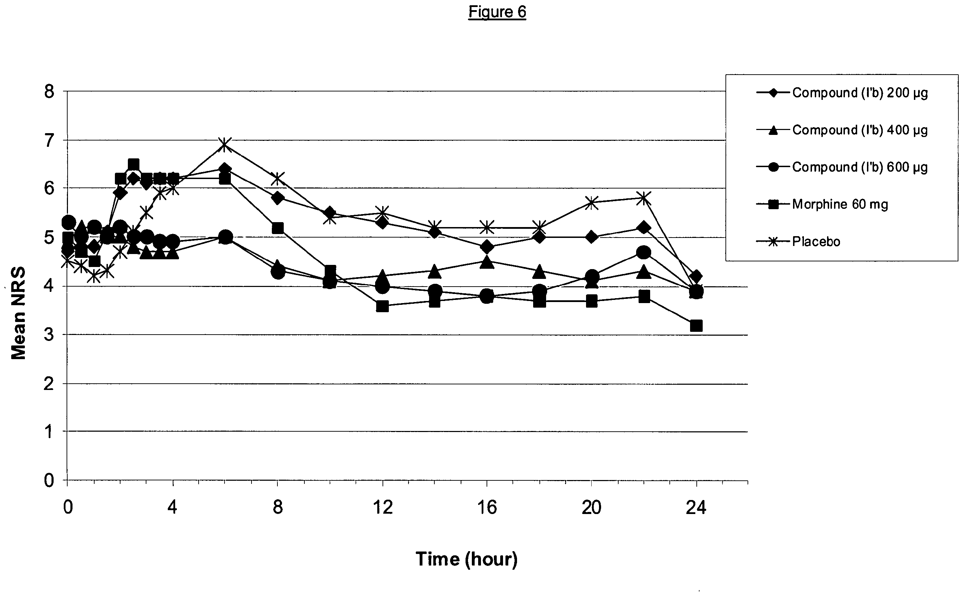

The primary efficacy assessment endpoint was the absolute pain intensity over a 24 hour period. Pain intensity was measured using an 11-point numerical rating scale (NRS). At each time point, patients were instructed to evaluate their current pain intensity relative to an 11-point numerical rating scale. A score of zero represented no pain and a score of 10 represented worst possible pain. Missing scheduled pain assessments for the patients were imputed with the last observation carried forward (LOCF). The resulting averaged NRS values over the 24 hour period are depicted in Figure 6.

Sum of pain intensity differences over different time periods were analyzed using an analysis of covariance (ANCOVA) model with factors for treatment and site and baseline pain intensity score (using the pain intensity NPRS score). Only subjects with non-missing baseline pain intensity were included. A summary of the analysis for the 2 to 10 hour period is presented in the table here below.

| | n | LS mean | SE | LS mean Δplacebo | SE | P-value |
|---|---|---|---|---|---|---|
| placebo | 45 | 49.13 | 2.85 | | | |
| compound (I'b) 200 µg | 52 | 46.05 | 2.78 | -3.08 | 3.49 | 0.3776 |
| compound (I'b) 400 µg | 47 | 35.28 | 2.81 | -13.85 | 3.57 | 0.0001 |
| compound (I'b) 600 µg | 55 | 35.15 | 2.67 | -13.98 | 3.45 | < 0.0001 |
| morphine, controlled-release 60 mg | 49 | 42.01 | 2.83 | -7.12 | 3.54 | 0.0454 |

| | | | | | | |
|---|---|---|---|---|---|---|
| LS mean: least squares means; SE: statistical error | | | | | | |

The resulting p-values are summarized in the following table:

| p-values (sum of pain intensity differences) | 2-6 h | 2-10 h | 2-12 h | 2-14 h | 2-18 h | 2-24 h |
|---|---|---|---|---|---|---|
| compound (I'b) 200 µg | 0.4514 | 0.3776 | 0.3387 | 0.3427 | 0.3205 | 0.2923 |
| compound (I'b) 400 µg | 0.0009 | 0.0001 | < 0.0001 | 0.0001 | 0.0005 | 0.0008 |
| compound (I'b) 600 µg | 0.0009 | < 0.0001 | < 0.0001 | < 0.0001 | < 0.0001 | 0.0001 |
| morphine, controlled-release 60 mg | 0.4664 | 0.0454 | 0.0084 | 0.0036 | 0.0014 | 0.0005 |

Accordingly, on the primary parameter, a statistically significant difference was observed between groups that had received a 400 µg or 600 µg dose of compound (I'b) and placebo groups, whereas no statistically significant difference was observed for groups that had received a 200 µg dose of compound (I'b).

The following two tables summarize the treatment emergent adverse events (TEAE(s)) experienced by the five treatment groups.

| | Placebo | compound (I'b) 200 µg | compound (I'b) 400 µg | compound (I'b) 600 µg | morphine 60 mg |
|---|---|---|---|---|---|
| subjects with TEAE(s) (n (%)) | 32 (68.1) | 37 (67.3) | 38 (77.6) | 48 (84.2) | 46 (92.0) |
| related (n (%)) | 17 (36.2) | 24 (43.6) | 32 (65.3) | 43 (75.4) | 42 (84.0) |
| serious (n (%)) | 1 (2.1) | 0 | 0 | 0 | 0 |
| total number of TEAE's (n) | 74 | 75 | 125 | 198 | 144 |
| related (n(%)) | 32 (43.2) | 37 (49.3) | 74 (59.2) | 146 (73.7) | 99 (68.8) |
| subjects with SAE's | 1 (2.1) | 0 | 0 | 0 | 0 |
| deaths | 0 | 0 | 0 | 0 | 0 |

| | | | | | |
|---|---|---|---|---|---|
| TEAE: treatment emergent adverse event; SAE: serious adverse event | | | | | |

| | Placebo | compound (I'b) 200 µg | compound (I'b) 400 µg | compound (I'b) 600 µg | morphine 60 mg |
|---|---|---|---|---|---|
| Nausea | 17.0 | 29.1 | 49.0 | 64.9 | 66.0 |
| Vomiting | 2.1 | 9.1 | 20.4 | 49.1 | 40.0 |
| Dizziness | 6.4 | 20.0 | 22.4 | 26.3 | 24.0 |
| Somnolence | 2.1 | 1.8 | 10.2 | 14.0 | 16.0 |
| ASAT increased | 2.1 | 1.8 | 6.1 | 1.8 | 2.0 |
| Hot flush | 0 | 1.8 | 4.1 | 7.0 | 4.0 |
| Pruritus | 0 | 0 | 6.1 | 3.5 | 2.0 |
| Hyperhidrosis | 0 | 0 | 0 | 5.3 | 6.0 |

| | | | | | |
|---|---|---|---|---|---|
| 100% = total number of subjects in corresponding treatment group; ASAT: aspartate aminotransferase | | | | | |

It becomes evident from the above tables that all four active treatments were well tolerated under these circumstances and the adverse events that showed up most frequently are in line with what can be expected from µ-opioid receptor agonists. For the patient group that had been treated with compound (I'b), the incidence of adverse events increased with the dose, and at a dose of 600 µg the incidence of adverse events was comparable to that of the morphine patient group.

### Prophetic examples

Prophetic examples of pharmaceutical dosage forms according to the invention are provided below. Their compositions are intended to be exemplary and it should be understood that the ingredients, the amount thereof and the procedure to obtain the dosage form may be varied.

In analogy to examples 1, 2 and 3, the following compositions (solid solutions) can be prepared in accordance with the general procedure:

**Prophetic example 1:**

| Ex. | API | polymer | weight ratio API : polymer | surfactant | content surfactant |
|---|---|---|---|---|---|
| P1-1-A-1 | + | Kollidon 90 | 1:8 | Tween 80 | 1 wt.-% |
| P1-1-A-2 | + | Kollidon 90 | 1:8 | Tween 80 | 2.5 wt.-% |
| P1-1-A-3 | + | Kollidon 90 | 1:8 | Tween 80 | 5 wt.-% |
| P1-1-A-4 | + | Kollidon 90 | 1:8 | Tween 80 | 7.5 wt.-% |
| P1-1-A-5 | + | Kollidon 90 | 1:8 | Tween 80 | 10 wt.-% |
| P1-1-B-1 | + | Kollidon 90 | 1:12 | Tween 80 | 1 wt.-% |
| P1-1-B-2 | + | Kollidon 90 | 1:12 | Tween 80 | 2.5 wt.-% |
| P1-1-B-3 | + | Kollidon 90 | 1:12 | Tween 80 | 5 wt.-% |
| P1-1-B-4 | + | Kollidon 90 | 1:12 | Tween 80 | 7.5 wt.-% |
| P1-1-B-5 | + | Kollidon 90 | 1:12 | Tween 80 | 10 wt.-% |
| P1-1-C-1 | + | Kollidon 90 | 1:16 | Tween 80 | 1 wt.-% |
| P1-1-C-2 | + | Kollidon 90 | 1:16 | Tween 80 | 2.5 wt.-% |
| P1-1-C-3 | + | Kollidon 90 | 1:16 | Tween 80 | 5 wt.-% |
| P1-1-C-4 | + | Kollidon 90 | 1:16 | Tween 80 | 7.5 wt.-% |
| P1-1-C-5 | + | Kollidon 90 | 1:16 | Tween 80 | 10 wt.-% |
| P1-1-D-1 | + | Kollidon 90 | 1:20 | Tween 80 | 1 wt.-% |
| P1-1-D-2 | + | Kollidon 90 | 1:20 | Tween 80 | 2.5 wt.-% |
| P1-1-D-3 | + | Kollidon 90 | 1:20 | Tween 80 | 5 wt.-% |
| P1-1-D-4 | + | Kollidon 90 | 1:20 | Tween 80 | 7.5 wt.-% |
| P1-1-D-5 | + | Kollidon 90 | 1:20 | Tween 80 | 10 wt.-% |
| P1-1-E-1 | + | Kollidon 90 | 1:24 | Tween 80 | 1 wt.-% |
| P1-1-E-2 | + | Kollidon 90 | 1:24 | Tween 80 | 2.5 wt.-% |
| P1-1-E-3 | + | Kollidon 90 | 1:24 | Tween 80 | 5 wt.-% |
| P1-1-E-4 | + | Kollidon 90 | 1:24 | Tween 80 | 7.5 wt.-% |
| P1-1-E-5 | + | Kollidon 90 | 1:24 | Tween 80 | 10 wt.-% |
| P1-2-A-1 | + | Kollidon 90 | 1:8 | Solutol HS 15 | 1 wt.-% |
| P1-2-A-2 | + | Kollidon 90 | 1:8 | Solutol HS 15 | 2.5 wt.-% |
| P1-2-A-3 | + | Kollidon 90 | 1:8 | Solutol HS 15 | 5 wt.-% |
| P1-2-A-4 | + | Kollidon 90 | 1:8 | Solutol HS 15 | 7.5 wt.-% |
| P1-2-A-5 | + | Kollidon 90 | 1:8 | Solutol HS 15 | 10 wt.-% |
| P1-2-B-1 | + | Kollidon 90 | 1:12 | Solutol HS 15 | 1 wt.-% |
| P1-2-B-2 | + | Kollidon 90 | 1:12 | Solutol HS 15 | 2.5 wt.-% |
| P1-2-B-3 | + | Kollidon 90 | 1:12 | Solutol HS 15 | 5 wt.-% |
| P1-2-B-4 | + | Kollidon 90 | 1:12 | Solutol HS 15 | 7.5 wt.-% |
| P1-2-B-5 | + | Kollidon 90 | 1:12 | Solutol HS 15 | 10 wt.-% |
| P1-2-C-1 | + | Kollidon 90 | 1:16 | Solutol HS 15 | 1 wt.-% |
| P1-2-C-2 | + | Kollidon 90 | 1:16 | Solutol HS 15 | 2.5 wt.-% |
| P1-2-C-3 | + | Kollidon 90 | 1:16 | Solutol HS 15 | 5 wt.-% |
| P1-2-C-4 | + | Kollidon 90 | 1:16 | Solutol HS 15 | 7.5 wt.-% |
| P1-2-C-5 | + | Kollidon 90 | 1:16 | Solutol HS 15 | 10 wt.-% |
| P1-2-D-1 | + | Kollidon 90 | 1:20 | Solutol HS 15 | 1 wt.-% |
| P1-2-D-2 | + | Kollidon 90 | 1:20 | Solutol HS 15 | 2.5 wt.-% |
| P1-2-D-3 | + | Kollidon 90 | 1:20 | Solutol HS 15 | 5 wt.-% |
| P1-2-D-4 | + | Kollidon 90 | 1:20 | Solutol HS 15 | 7.5 wt.-% |
| P1-2-D-5 | + | Kollidon 90 | 1:20 | Solutol HS 15 | 10 wt.-% |
| P1-2-E-1 | + | Kollidon 90 | 1:24 | Solutol HS 15 | 1 wt.-% |
| P1-2-E-2 | + | Kollidon 90 | 1:24 | Solutol HS 15 | 2.5 wt.-% |
| P1-2-E-3 | + | Kollidon 90 | 1:24 | Solutol HS 15 | 5 wt.-% |
| P1-2-E-4 | + | Kollidon 90 | 1:24 | Solutol HS 15 | 7.5 wt.-% |
| P1-2-E-5 | + | Kollidon 90 | 1:24 | Solutol HS 15 | 10 wt.-% |
| P1-3-A-1 | + | Kollidon 90 | 1:8 | Tween 60 | 1 wt.-% |
| P1-3-A-2 | + | Kollidon 90 | 1:8 | Tween 60 | 2.5 wt.-% |
| P1-3-A-3 | + | Kollidon 90 | 1:8 | Tween 60 | 5 wt.-% |
| P1-3-A-4 | + | Kollidon 90 | 1:8 | Tween 60 | 7.5 wt.-% |
| P1-3-A-5 | + | Kollidon 90 | 1:8 | Tween 60 | 10 wt.-% |
| P1-3-B-1 | + | Kollidon 90 | 1:12 | Tween 60 | 1 wt.-% |
| P1-3-B-2 | + | Kollidon 90 | 1:12 | Tween 60 | 2.5 wt.-% |
| P1-3-B-3 | + | Kollidon 90 | 1:12 | Tween 60 | 5 wt.-% |
| P1-3-B-4 | + | Kollidon 90 | 1:12 | Tween 60 | 7.5 wt.-% |
| P1-3-B-5 | + | Kollidon 90 | 1:12 | Tween 60 | 10 wt.-% |
| P1-3-C-1 | + | Kollidon 90 | 1:16 | Tween 60 | 1 wt.-% |
| P1-3-C-2 | + | Kollidon 90 | 1:16 | Tween 60 | 2.5 wt.-% |
| P1-3-C-3 | + | Kollidon 90 | 1:16 | Tween 60 | 5 wt.-% |
| P1-3-C-4 | + | Kollidon 90 | 1:16 | Tween 60 | 7.5 wt.-% |
| P1-3-C-5 | + | Kollidon 90 | 1:16 | Tween 60 | 10 wt.-% |
| P1-3-D-1 | + | Kollidon 90 | 1:20 | Tween 60 | 1 wt.-% |
| P1-3-D-2 | + | Kollidon 90 | 1:20 | Tween 60 | 2.5 wt.-% |
| P1-3-D-3 | + | Kollidon 90 | 1:20 | Tween 60 | 5 wt.-% |
| P1-3-D-4 | + | Kollidon 90 | 1:20 | Tween 60 | 7.5 wt-.% |
| P1-3-D-5 | + | Kollidon 90 | 1:20 | Tween 60 | 10 wt.-% |
| P1-3-E-1 | + | Kollidon 90 | 1:24 | Tween 60 | 1 wt.-% |
| P1-3-E-2 | + | Kollidon 90 | 1:24 | Tween 60 | 2.5 wt.-% |
| P1-3-E-3 | + | Kollidon 90 | 1:24 | Tween 60 | 5 wt.-% |
| P1-3-E-4 | + | Kollidon 90 | 1:24 | Tween 60 | 7.5 wt.-% |
| P1-3-E-5 | + | Kollidon 90 | 1:24 | Tween 60 | 10 wt.-% |
| P1-4-A-1 | + | Kollidon 90 | 1:8 | Myrj 51 | 1 wt.-% |
| P1-4-A-2 | + | Kollidon 90 | 1:8 | Myrj 51 | 2.5 wt.-% |
| P1-4-A-3 | + | Kollidon 90 | 1:8 | Myrj 51 | 5 wt.-% |
| P1-4-A-4 | + | Kollidon 90 | 1:8 | Myrj 51 | 7.5 wt.-% |
| P1-4-A-5 | + | Kollidon 90 | 1:8 | Myrj 51 | 10 wt.-% |
| P1-4-B-1 | + | Kollidon 90 | 1:12 | Myrj 51 | 1 wt.-% |
| P1-4-B-2 | + | Kollidon 90 | 1:12 | Myrj 51 | 2.5 wt.-% |
| P1-4-B-3 | + | Kollidon 90 | 1:12 | Myrj 51 | 5 wt.-% |
| P1-4-B-4 | + | Kollidon 90 | 1:12 | Myrj 51 | 7.5 wt.-% |
| P1-4-B-5 | + | Kollidon 90 | 1:12 | Myrj 51 | 10 wt.-% |
| P1-4-C-1 | + | Kollidon 90 | 1:16 | Myrj 51 | 1 wt.-% |
| P1-4-C-2 | + | Kollidon 90 | 1:16 | Myrj 51 | 2.5 wt.-% |
| P1-4-C-3 | + | Kollidon 90 | 1:16 | Myrj 51 | 5 wt.-% |
| P1-4-C-4 | + | Kollidon 90 | 1:16 | Myrj 51 | 7.5 wt.-% |
| P1-4-C-5 | + | Kollidon 90 | 1:16 | Myrj 51 | 10 wt.-% |
| P1-4-D-1 | + | Kollidon 90 | 1:20 | Myrj 51 | 1 wt.-% |
| P1-4-D-2 | + | Kollidon 90 | 1:20 | Myrj 51 | 2.5 wt.-% |
| P1-4-D-3 | + | Kollidon 90 | 1:20 | Myrj 51 | 5 wt.-% |
| P1-4-D-4 | + | Kollidon 90 | 1:20 | Myrj 51 | 7.5 wt.-% |
| P1-4-D-5 | + | Kollidon 90 | 1:20 | Myrj 51 | 10 wt.-% |
| P1-4-E-1 | + | Kollidon 90 | 1:24 | Myrj 51 | 1 wt.-% |
| P1-4-E-2 | + | Kollidon 90 | 1:24 | Myrj 51 | 2.5 wt.-% |
| P1-4-E-3 | + | Kollidon 90 | 1:24 | Myrj 51 | 5 wt.-% |
| P1-4-E-4 | + | Kollidon 90 | 1:24 | Myrj 51 | 7.5 wt.-% |
| P1-4-E-5 | + | Kollidon 90 | 1:24 | Myrj 51 | 10 wt.-% |
| P1-5-A-1 | + | Kollidon 90 | 1:8 | Brij 98 | 1 wt.-% |
| P1-5-A-2 | + | Kollidon 90 | 1:8 | Brij 98 | 2.5 wt.-% |
| P1-5-A-3 | + | Kollidon 90 | 1:8 | Brij 98 | 5 wt.-% |
| P1-5-A-4 | + | Kollidon 90 | 1:8 | Brij 98 | 7.5 wt.-% |
| P1-5-A-5 | + | Kollidon 90 | 1:8 | Brij 98 | 10 wt.-% |
| P1-5-B-1 | + | Kollidon 90 | 1:12 | Brij 98 | 1 wt.-% |
| P1-5-B-2 | + | Kollidon 90 | 1:12 | Brij 98 | 2.5 wt.-% |
| P1-5-B-3 | + | Kollidon 90 | 1:12 | Brij 98 | 5 wt.-% |
| P1-5-B-4 | + | Kollidon 90 | 1:12 | Brij 98 | 7.5 wt.-% |
| P1-5-B-5 | + | Kollidon 90 | 1:12 | Brij 98 | 10 wt.-% |
| P1-5-C-1 | + | Kollidon 90 | 1:16 | Brij 98 | 1 wt.-% |
| P1-5-C-2 | + | Kollidon 90 | 1:16 | Brij 98 | 2.5 wt.-% |
| P1-5-C-3 | + | Kollidon 90 | 1:16 | Brij 98 | 5 wt.-% |
| P1-5-C-4 | + | Kollidon 90 | 1:16 | Brij 98 | 7.5 wt.-% |
| P1-5-C-5 | + | Kollidon 90 | 1:16 | Brij 98 | 10 wt.-% |
| P1-5-D-1 | + | Kollidon 90 | 1:20 | Brij 98 | 1 wt.-% |
| P1-5-D-2 | + | Kollidon 90 | 1:20 | Brij 98 | 2.5 wt.-% |
| P1-5-D-3 | + | Kollidon 90 | 1:20 | Brij 98 | 5 wt.-% |
| P1-5-D-4 | + | Kollidon 90 | 1:20 | Brij 98 | 7.5 wt.-% |
| P1-5-D-5 | + | Kollidon 90 | 1:20 | Brij 98 | 10 wt.-% |
| P1-5-E-1 | + | Kollidon 90 | 1:24 | Brij 98 | 1 wt.-% |
| P1-5-E-2 | + | Kollidon 90 | 1:24 | Brij 98 | 2.5 wt.-% |
| P1-5-E-3 | + | Kollidon 90 | 1:24 | Brij 98 | 5 wt.-% |
| P1-5-E-4 | + | Kollidon 90 | 1:24 | Brij 98 | 7.5 wt.-% |
| P1-5-E-5 | + | Kollidon 90 | 1:24 | Brij 98 | 10 wt.-% |

**Prophetic example 2:**

| Ex. | API | polymer | weight ratio API : polymer | surfactant | content surfactant |
|---|---|---|---|---|---|
| P2-1-A-1 | + | Kollidon VA64 | 1:8 | Tween 80 | 1 wt.-% |
| P2-1-A-2 | + | Kollidon VA64 | 1:8 | Tween 80 | 2.5 wt.-% |
| P2-1-A-3 | + | Kollidon VA64 | 1:8 | Tween 80 | 5 wt.-% |
| P2-1-A-4 | + | Kollidon VA64 | 1:8 | Tween 80 | 7.5 wt.-% |
| P2-1-A-5 | + | Kollidon VA64 | 1:8 | Tween 80 | 10 wt.-% |
| P2-1-B-1 | + | Kollidon VA64 | 1:12 | Tween 80 | 1 wt.-% |
| P2-1-B-2 | + | Kollidon VA64 | 1:12 | Tween 80 | 2.5 wt.-% |
| P2-1-B-3 | + | Kollidon VA64 | 1:12 | Tween 80 | 5 wt.-% |
| P2-1-B-4 | + | Kollidon VA64 | 1:12 | Tween 80 | 7.5 wt.-% |
| P2-1-B-5 | + | Kollidon VA64 | 1:12 | Tween 80 | 10 wt.-% |
| P2-1-C-1 | + | Kollidon VA64 | 1:16 | Tween 80 | 1 wt.-% |
| P2-1-C-2 | + | Kollidon VA64 | 1:16 | Tween 80 | 2.5 wt.-% |
| P2-1-C-3 | + | Kollidon VA64 | 1:16 | Tween 80 | 5 wt.-% |
| P2-1-C-4 | + | Kollidon VA64 | 1:16 | Tween 80 | 7.5 wt.-% |
| P2-1-C-5 | + | Kollidon VA64 | 1:16 | Tween 80 | 10 wt.-% |
| P2-1-D-1 | + | Kollidon VA64 | 1:20 | Tween 80 | 1 wt.-% |
| P2-1-D-2 | + | Kollidon VA64 | 1:20 | Tween 80 | 2.5 wt.-% |
| P2-1-D-3 | + | Kollidon VA64 | 1:20 | Tween 80 | 5 wt.-% |
| P2-1-D-4 | + | Kollidon VA64 | 1:20 | Tween 80 | 7.5 wt.-% |
| P2-1-D-5 | + | Kollidon VA64 | 1:20 | Tween 80 | 10 wt.-% |
| P2-1-E-1 | + | Kollidon VA64 | 1:24 | Tween 80 | 1 wt.-% |
| P2-1-E-2 | + | Kollidon VA64 | 1:24 | Tween 80 | 2.5 wt.-% |
| P2-1-E-3 | + | Kollidon VA64 | 1:24 | Tween 80 | 5 wt.-% |
| P2-1-E-4 | + | Kollidon VA64 | 1:24 | Tween 80 | 7.5 wt.-% |
| P2-1-E-5 | + | Kollidon VA64 | 1:24 | Tween 80 | 10 wt.-% |
| P2-2-A-1 | + | Kollidon VA64 | 1:8 | Solutol HS 15 | 1 wt.-% |
| P2-2-A-2 | + | Kollidon VA64 | 1:8 | Solutol HS 15 | 2.5 wt.-% |
| P2-2-A-3 | + | Kollidon VA64 | 1:8 | Solutol HS 15 | 5 wt.-% |
| P2-2-A-4 | + | Kollidon VA64 | 1:8 | Solutol HS 15 | 7.5 wt.-% |
| P2-2-A-5 | + | Kollidon VA64 | 1:8 | Solutol HS 15 | 10 wt.-% |
| P2-2-B-1 | + | Kollidon VA64 | 1:12 | Solutol HS 15 | 1 wt.-% |
| P2-2-B-2 | + | Kollidon VA64 | 1:12 | Solutol HS 15 | 2.5 wt.-% |
| P2-2-B-3 | + | Kollidon VA64 | 1:12 | Solutol HS 15 | 5 wt.-% |
| P2-2-B-4 | + | Kollidon VA64 | 1:12 | Solutol HS 15 | 7.5 wt.-% |
| P2-2-B-5 | + | Kollidon VA64 | 1:12 | Solutol HS 15 | 10 wt.-% |
| P2-2-C-1 | + | Kollidon VA64 | 1:16 | Solutol HS 15 | 1 wt.% |
| P2-2-C-2 | + | Kollidon VA64 | 1:16 | Solutol HS 15 | 2.5 wt.-% |
| P2-2-C-3 | + | Kollidon VA64 | 1:16 | Solutol HS 15 | 5 wt.-% |
| P2-2-C-4 | + | Kollidon VA64 | 1:16 | Solutol HS 15 | 7.5 wt.-% |
| P2-2-C-5 | + | Kollidon VA64 | 1:16 | Solutol HS 15 | 10 wt.% |
| P2-2-D-1 | + | Kollidon VA64 | 1:20 | Solutol HS 15 | 1 wt.-% |
| P2-2-D-2 | + | Kollidon VA64 | 1:20 | Solutol HS 15 | 2.5 wt.-% |
| P2-2-D-3 | + | Kollidon VA64 | 1:20 | Solutol HS 15 | 5 wt.-% |
| P2-2-D-4 | + | Kollidon VA64 | 1:20 | Solutol HS 15 | 7.5 wt.-% |
| P2-2-D-5 | + | Kollidon VA64 | 1:20 | Solutol HS 15 | 10 wt.-% |
| P2-2-E-1 | + | Kollidon VA64 | 1:24 | Solutol HS 15 | 1 wt.-% |
| P2-2-E-2 | + | Kollidon VA64 | 1:24 | Solutol HS 15 | 2.5 wt.-% |
| P2-2-E-3 | + | Kollidon VA64 | 1:24 | Solutol HS 15 | 5 wt.-% |
| P2-2-E-4 | + | Kollidon VA64 | 1:24 | Solutol HS 15 | 7.5 wt.-% |
| P2-2-E-5 | + | Kollidon VA64 | 1:24 | Solutol HS 15 | 10 wt.-% |
| P2-3-A-1 | + | Kollidon VA64 | 1:8 | Pluronic F127 | 1 wt.-% |
| P2-3-A-2 | + | Kollidon VA64 | 1:8 | Pluronic F127 | 2.5 wt.-% |
| P2-3-A-3 | + | Kollidon VA64 | 1:8 | Pluronic F127 | 5 wt.-% |
| P2-3-A-4 | + | Kollidon VA64 | 1:8 | Pluronic F127 | 7.5 wt.-% |
| P2-3-A-5 | + | Kollidon VA64 | 1:8 | Pluronic F127 | 10 wt.-% |
| P2-3-B-1 | + | Kollidon VA64 | 1:12 | Pluronic F127 | 1 wt.-% |
| P2-3-B-2 | + | Kollidon VA64 | 1:12 | Pluronic F127 | 2.5 wt.-% |
| P2-3-B-3 | + | Kollidon VA64 | 1:12 | Pluronic F127 | 5wt.-% |
| P2-3-B-4 | + | Kollidon VA64 | 1:12 | Pluronic F127 | 7.5 wt.-% |
| P2-3-B-5 | + | Kollidon VA64 | 1:12 | Pluronic F127 | 10 wt.-% |
| P2-3-C-1 | + | Kollidon VA64 | 1:16 | Pluronic F127 | 1 wt.-% |
| P2-3-C-2 | + | Kollidon VA64 | 1:16 | Pluronic F127 | 2.5 wt.-% |
| P2-3-C-3 | + | Kollidon VA64 | 1:16 | Pluronic F127 | 5 wt.-% |
| P2-3-C-4 | + | Kollidon VA64 | 1:16 | Pluronic F127 | 7.5 wt.-% |
| P2-3-C-5 | + | Kollidon VA64 | 1:16 | Pluronic F127 | 10 wt.-% |
| P2-3-D-1 | + | Kollidon VA64 | 1:20 | Pluronic F127 | 1 wt.-% |
| P2-3-D-2 | + | Kollidon VA64 | 1:20 | Pluronic F127 | 2.5 wt.-% |
| P2-3-D-3 | + | Kollidon VA64 | 1:20 | Pluronic F127 | 5 wt.-% |
| P2-3-D-4 | + | Kollidon VA64 | 1:20 | Pluronic F127 | 7.5 wt.-% |
| P2-3-D-5 | + | Kollidon VA64 | 1:20 | Pluronic F127 | 10 wt.-% |
| P2-3-E-1 | + | Kollidon VA64 | 1:24 | Pluronic F127 | 1 wt.-% |
| P2-3-E-2 | + | Kollidon VA64 | 1:24 | Pluronic F127 | 2.5 wt.-% |
| P2-3-E-3 | + | Kollidon VA64 | 1:24 | Pluronic F127 | 5 wt.-% |
| P2-3-E-4 | + | Kollidon VA64 | 1:24 | Pluronic F127 | 7.5 wt.-% |
| P2-3-E-5 | + | Kollidon VA64 | 1:24 | Pluronic F127 | 10 wt.-% |
| P2-4-A-1 | + | Kollidon VA64 | 1:8 | Myrj 51 | 1 wt.-% |
| P2-4-A-2 | + | Kollidon VA64 | 1:8 | Myrj 51 | 2.5 wt.-% |
| P2-4-A-3 | + | Kollidon VA64 | 1:8 | Myrj 51 | 5 wt.-% |
| P2-4-A-4 | + | Kollidon VA64 | 1:8 | Myrj 51 | 7.5 wt.-% |
| P2-4-A-5 | + | Kollidon VA64 | 1:8 | Myrj 51 | 10 wt.-% |
| P2-4-B-1 | + | Kollidon VA64 | 1:12 | Myrj 51 | 1 wt.-% |
| P2-4-B-2 | + | Kollidon VA64 | 1:12 | Myrj 51 | 2.5 wt.-% |
| P2-4-B-3 | + | Kollidon VA64 | 1:12 | Myrj 51 | 5 wt.-% |
| P2-4-B-4 | + | Kollidon VA64 | 1:12 | Myrj 51 | 7.5 wt.-% |
| P2-4-B-5 | + | Kollidon VA64 | 1:12 | Myrj 51 | 10 wt.-% |
| P2-4-C-1 | + | Kollidon VA64 | 1:16 | Myrj 51 | 1 wt.-% |
| P2-4-C-2 | + | Kollidon VA64 | 1:16 | Myrj 51 | 2.5 wt.-% |
| P2-4-C-3 | + | Kollidon VA64 | 1:16 | Myrj 51 | 5 wt.-% |
| P2-4-C-4 | + | Kollidon VA64 | 1:16 | Myrj 51 | 7.5 wt.-% |
| P2-4-C-5 | + | Kollidon VA64 | 1:16 | Myrj 51 | 10 wt.-% |
| P2-4-D-1 | + | Kollidon VA64 | 1:20 | Myrj 51 | 1 wt.-% |
| P2-4-D-2 | + | Kollidon VA64 | 1:20 | Myrj 51 | 2.5 wt.-% |
| P2-4-D-3 | + | Kollidon VA64 | 1:20 | Myrj 51 | 5 wt.-% |
| P2-4-D-4 | + | Kollidon VA64 | 1:20 | Myrj 51 | 7.5 wt.-% |
| P2-4-D-5 | + | Kollidon VA64 | 1:20 | Myrj 51 | 10 wt.-% |
| P2-4-E-1 | + | Kollidon VA64 | 1:24 | Myrj 51 | 1 wt.-% |
| P2-4-E-2 | + | Kollidon VA64 | 1:24 | Myrj 51 | 2.5 wt.-% |
| P2-4-E-3 | + | Kollidon VA64 | 1:24 | Myrj 51 | 5 wt.-% |
| P2-4-E-4 | + | Kollidon VA64 | 1:24 | Myrj 51 | 7.5 wt.-% |
| P2-4-E-5 | + | Kollidon VA64 | 1:24 | Myrj 51 | 10 wt.-% |
| P2-5-A-1 | + | Kollidon VA64 | 1:8 | Brij 98 | 1 wt.-% |
| P2-5-A-2 | + | Kollidon VA64 | 1:8 | Brij 98 | 2.5 wt.-% |
| P2-5-A-3 | + | Kollidon VA64 | 1:8 | Brij 98 | 5 wt.-% |
| P2-5-A-4 | + | Kollidon VA64 | 1:8 | Brij 98 | 7.5 wt.-% |
| P2-5-A-5 | + | Kollidon VA64 | 1:8 | Brij 98 | 10 wt.-% |
| P2-5-B-1 | + | Kollidon VA64 | 1:12 | Brij 98 | 1 wt.-% |
| P2-5-B-2 | + | Kollidon VA64 | 1:12 | Brij 98 | 2.5 wt.-% |
| P2-5-B-3 | + | Kollidon VA64 | 1:12 | Brij 98 | 5 wt.-% |
| P2-5-B-4 | + | Kollidon VA64 | 1:12 | Brij 98 | 7.5 wt.-% |
| P2-5-B-5 | + | Kollidon VA64 | 1:12 | Brij 98 | 10 wt.-% |
| P2-5-C-1 | + | Kollidon VA64 | 1:16 | Brij 98 | 1 wt.-% |
| P2-5-C-2 | + | Kollidon VA64 | 1:16 | Brij 98 | 2.5 wt.-% |
| P2-5-C-3 | + | Kollidon VA64 | 1:16 | Brij 98 | 5 wt.-% |
| P2-5-C-4 | + | Kollidon VA64 | 1:16 | Brij 98 | 7.5 wt.-% |
| P2-5-C-5 | + | Kollidon VA64 | 1:16 | Brij 98 | 10 wt.-% |
| P2-5-D-1 | + | Kollidon VA64 | 1:20 | Brij 98 | 1 wt.-% |
| P2-5-D-2 | + | Kollidon VA64 | 1:20 | Brij 98 | 2.5 wt.-% |
| P2-5-D-3 | + | Kollidon VA64 | 1:20 | Brij 98 | 5 wt.-% |
| P2-5-D-4 | + | Kollidon VA64 | 1:20 | Brij 98 | 7.5 wt.-% |
| P2-5-D-5 | + | Kollidon VA64 | 1:20 | Brij 98 | 10 wt.-% |
| P2-5-E-1 | + | Kollidon VA64 | 1:24 | Brij 98 | 1 wt.-% |
| P2-5-E-2 | + | Kollidon VA64 | 1:24 | Brij 98 | 2.5 wt.-% |
| P2-5-E-3 | + | Kollidon VA64 | 1:24 | Brij 98 | 5 wt.-% |
| P2-5-E-4 | + | Kollidon VA64 | 1:24 | Brij 98 | 7.5 wt.-% |
| P2-5-E-5 | + | Kollidon VA64 | 1:24 | Brij 98 | 10 wt.-% |

## Claims

1. A pharmaceutical dosage form for administration twice daily, once daily or less frequently,
which contains a pharmacologically active agent according to general formula (I) 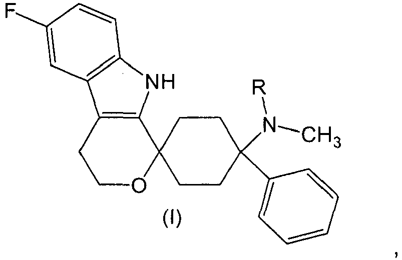 wherein R is -H or -CH₃,
or a physiologically acceptable salt thereof;
which releases in accordance with Ph. Eur. under *in vitro* conditions in 900 mL artificial gastric juice at pH 1.2 and 37 ± 0.5°C after 30 minutes according to the paddle method with sinker at 100 rpm at least 50 wt.-% of the pharmacologically active agent, based on the total amount of the pharmacologically active agent originally contained in the pharmaceutical dosage form; and
which comprises a solid polymeric matrix material in which the pharmacologically active agent according to general formula (I) is dispersed and immobilized in an amorphous or semi-amorphous state, and wherein the polymer is selected from the group consisting of polyvinylpyrrolidone, vinylpyrrolidone-polyvinylacetate copolymers, and any combinations thereof; and
wherein the relative weight ratio of the polymer related to the pharmacologically active agent according to general formula (I) is at least 6:1.

2. The pharmaceutical dosage form according to claim 1, which further comprises a surfactant.

3. The pharmaceutical dosage form according to claim 2, wherein
- the surfactant has a HLB value of at least 10; and/or
- the content of the surfactant is at least 0.001 wt.-%, based on the total weight of the pharmaceutical dosage form.

4. The pharmaceutical dosage form according to claim 2 or 3, wherein the surfactant is selected from the group consisting of polyoxyethylene fatty acid esters, partial fatty acid esters of polyoxyethylenesorbitan, and sulfuric acid esters.

5. The pharmaceutical dosage form according to any of the preceding claims, wherein the pharmacologically active agent according to general formula (I) has a stereochemistry according to general formula (I') 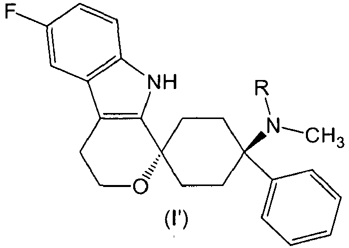 wherein R is defined as in claim 1.

6. The pharmaceutical dosage form according to any of the preceding claims, wherein the pharmacologically active agent according to general formula (I) is (1r,4r)-6'-fluoro-N,N-dimethyl-4-phenyl-4',9'-dihydro-3'H-spiro[cyclohexane-1,1'-pyrano[3,4,b]indol]-4-amine, (1r,4r)-6'-fluoro-N-methyl-4-phenyl-4',9'-dihydro-3'H-spiro[cyclohexane-1,1'-pyrano-[3,4,b]indol]-4-amine, or a physiologically acceptable salt thereof.

7. The pharmaceutical dosage form according to any of the preceding claims, which releases under *in vitro* conditions in 900 mL artificial gastric juice at pH 1.2 after 30 minutes at least 80 wt.-% of the pharmacologically active agent according to general formula (I), based on the total amount of the pharmacologically active agent according to general formula (I) originally contained in the dosage form.

8. The pharmaceutical dosage form according to any of the preceding claims, which contains the pharmacologically active agent according to general formula (I) in a dose of from 10 µg to 50 µg or of from 300 µg to 500 µg.

9. The pharmaceutical dosage form according to any of the preceding claims for use in the treatment of pain.

10. The pharmaceutical dosage form according to claim 9, wherein the pain is selected from acute, visceral, neuropathic or chronic pain.

## Patentansprüche

1. Pharmazeutische Darreichungsform zur zweimal täglichen, einmal täglichen oder weniger häufigen Verabreichung, enthaltend einen pharmakologisch wirksamen Stoff gemäß der allgemeinen Formel (I) 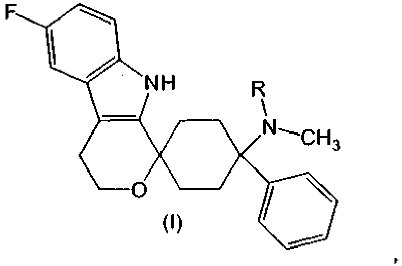 wobei R für -H oder -CH₃
oder ein physiologisch akzeptables Salz davon steht;
welche gemäß Ph. Eur. unter In-vitro-Bedingungen in 900 ml künstlichem Magensaft bei einem pH von 1,2 und 37 ± 0,5 °C nach 30 Minuten nach Paddelrührerverfahren bei 100 rpm mindestens 50 Gew.-% des pharmakologisch wirksamen Stoffs freisetzt, was auf der Gesamtmenge des pharmakologisch wirksamen Stoffs beruht, die ursprünglich in der pharmazeutischen Darreichungsform enthalten ist; und
welche ein festes Polymermatrix-Material enthält, in dem der pharmakologisch wirksame Stoff gemäß der allgemeinen Formel (I) in einem amorphen oder halbamorphen Zustand dispergiert und immobilisiert ist, und wobei das Polymer aus der Gruppe ausgewählt ist, die aus Polyvinylpyrrolidon, Vinylpyrrolidon/Polyvinylacetat-Copolymeren und jeder beliebigen Kombination daraus besteht; und
wobei das relative Gewichtsverhältnis des Polymers im Verhältnis zu dem pharmakologisch wirksamen Stoff gemäß der allgemeinen Formel (I) zumindest 6:1 beträgt.

2. Pharmazeutische Darreichungsform nach Anspruch 1, ferner umfassend eine oberflächenaktive Substanz.

3. Pharmazeutische Darreichungsform nach Anspruch 2, wobei
- die oberflächenaktive Substanz einen HLB-Wert von mindestens 10 aufweist; und/oder
- der Gehalt der oberflächenaktiven Substanz auf der Grundlage des Gesamtgewichts der pharmazeutischen Darreichungsform mindestens 0,001 Gew.-% beträgt.

4. Pharmazeutische Darreichungsform nach Anspruch 2 oder 3, wobei die oberflächenaktive Substanz ausgewählt wird aus der Gruppe bestehend aus Polyoxyethylenfettsäureestern, partiellen Fettsäureestern von Polyoxyethylensorbitan und Schwefelsäureestern.

5. Pharmazeutische Darreichungsform nach einem der vorhergehenden Ansprüche, wobei der pharmakologisch wirksame Stoff gemäß der allgemeinen Formel (I) eine Stereochemie gemäß der allgemeinen Formel (I') aufweist 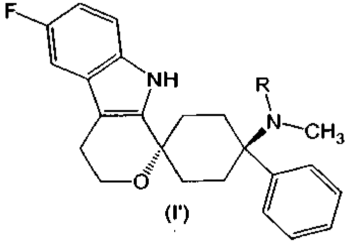 wobei R wie in Anspruch 1 definiert ist.

6. Pharmazeutische Darreichungsform nach einem der vorhergehenden Ansprüche, wobei der pharmazeutisch wirksame Stoff gemäß der allgemeinen Formel (I) (1r,4r)-6'-Fluor-N,N-dimethyl-4-phenyl-4',9'-dihydro-3'H-spiro[cyclohexan-1,1'-pyrano[3,4,b]indol]-4-amin, (1r,4r)-6'-Fluor-N-methyl-4-phenyl-4',9'-dihydro-3'H-spiro[cyclohexan-1,1'-pyrano-[3,4,b]indol]-4-amin oder ein physiologisch akzeptables Salz davon ist.

7. Pharmazeutische Darreichungsform nach einem der vorhergehenden Ansprüche, welche unter In-vitro-Bedingungen in 900 ml künstlichem Magensaft bei einem pH von 1,2 nach 30 Minuten mindestens 80 Gew.-% des pharmakologisch wirksamen Stoffs gemäß der allgemeinen Formel (I), auf der Grundlage der Gesamtmenge des pharmakologisch wirksamen Stoffs gemäß der allgemeinen Formel (I), die ursprünglich in der Darreichungsform enthalten ist, freisetzt.

8. Pharmazeutische Darreichungsform nach einem der vorhergehenden Ansprüche, welche den pharmakologisch wirksamen Stoff gemäß der allgemeinen Formel (I) in einer Dosis von 10 µg bis 50 µg oder von 300 µg bis 500 µg enthält.

9. Pharmazeutische Darreichungsform nach einem der vorhergehenden Ansprüche zur Verwendung bei der Behandlung von Schmerzen.

10. Pharmazeutische Darreichungsform nach Anspruch 9, wobei die Schmerzen ausgewählt sind aus akuten, viszeralen, neuropathischen oder chronischen Schmerzen.

## Revendications

1. Forme galénique pharmaceutique pour une administration deux fois par jour, une fois par jour ou moins fréquemment
qui contient un agent pharmacologiquement actif selon la formule générale (I) 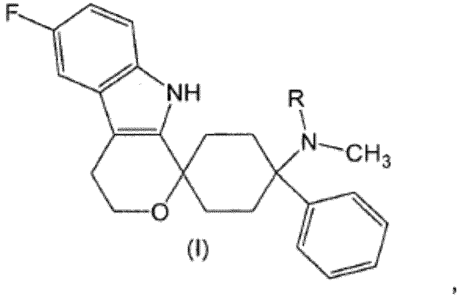 dans laquelle R représente -H ou -CH₃,
ou l'un de ses sels physiologiquement acceptables ; qui libère conformément à la Pharmacopée Européenne dans des conditions *in vitro* dans 900 ml de suc gastrique artificiel à pH 1,2 et 37 ± 0,5°C après 30 minutes selon le procédé à palette avec un lest à 100 tr/min au moins 50 % en poids de l'agent pharmacologiquement actif, en se basant sur la quantité totale de l'agent pharmacologiquement actif contenu à l'origine dans la forme galénique pharmaceutique ; et
qui comprend un matériau de matrice polymère solide dans lequel l'agent pharmacologiquement actif selon la formule générale (I) est dispersé et immobilisé dans un état amorphe ou semi-amorphe, et dans lequel le polymère est choisi dans le groupe constitué de polyvinylpyrrolidone, copolymères de vinylpyrrolidone-polyacétate de vinyle, et l'une quelconque de leurs combinaisons ; et
dans laquelle le rapport pondéral du polymère par rapport à l'agent pharmacologiquement actif selon la formule générale (I) est d'au moins 6/1.

2. Forme galénique pharmaceutique selon la revendication 1, qui comprend en outre un tensioactif.

3. Forme galénique pharmaceutique selon la revendication 2, dans laquelle
- le tensioactif a une valeur d'EHL d'au moins 10 ; et/ou
- la teneur en surfactant est d'au moins 0,001 % en poids, en se basant sur le poids total de la forme galénique pharmaceutique.

4. Forme galénique pharmaceutique selon la revendication 2 ou 3, dans laquelle le tensioactif est choisi dans le groupe constitué d'esters d'acides gras poly-oxyéthylénés, d'esters d'acides gras partiels de poly-oxyéthylène sorbitane, et d'esters d'acide sulfurique.

5. Forme galénique pharmaceutique selon l'une quelconque des revendications précédentes, dans laquelle l'agent pharmacologiquement actif selon la formule générale (I) présente une stéréochimie selon la formule générale (I') 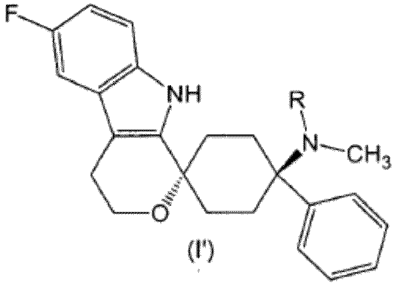 dans laquelle R est tel que défini dans la revendication 1.

6. Forme galénique pharmaceutique selon l'une quelconque des revendications précédentes, dans laquelle l'agent pharmacologiquement actif selon la formule (I) est la (1r,4r)-6'-fluoro-N,N-diméthyl-4-phényl-4',9'-dihydro-3'H-spiro[cyclohexane-1,1'-pyrano[3,4,b]indol]-4-amine, la (1r,4r)-6'-fluoro-N-méthyl-4-phényl-4',9'-dihydro-3'H-spiro-[cyclohexane-1,1'-pyrano-[3,4,b]indol]-4-amine, ou l'un de leurs sels physiologiquement acceptables.

7. Forme galénique pharmaceutique selon l'une quelconque des revendications précédentes qui libère dans des conditions *in vitro* dans 900 ml de suc gastrique artificiel à pH 1,2 après 30 minutes au moins 80 % en poids de l'agent pharmacologiquement actif selon la formule générale (I), en se basant sur la quantité totale de l'agent pharmacologiquement actif selon la formule générale (I) contenu à l'origine dans la forme galénique.

8. Forme galénique pharmaceutique selon l'une quelconque des revendications précédentes, qui contient l'agent pharmacologiquement actif selon la formule générale (I) dans une dose de 10 µg à 50 µg ou de 300 µg à 500 µg.

9. Forme galénique pharmaceutique selon l'une quelconque des revendications précédentes destinée à une utilisation dans le traitement de la douleur.

10. Forme galénique pharmaceutique selon la revendication 9, où la douleur est choisie parmi la douleur aiguë, viscérale, neuropathique ou chronique.
